(19) Europäisches Patentamt
European Patent Office
Office européen des brevets

(11) **EP 4 671 763 A1**

(12) **EUROPEAN PATENT APPLICATION**
published in accordance with Art. 153(4) EPC

(43) Date of publication:
**31.12.2025 Bulletin 2026/01**

(21) Application number: **24780097.2**

(22) Date of filing: **25.03.2024**

(51) International Patent Classification (IPC):
**G01N 33/543** (2006.01) **G01N 21/78** (2006.01)
**G01N 33/553** (2006.01)

(52) Cooperative Patent Classification (CPC):
**G01N 21/78; G01N 33/543; G01N 33/553**

(86) International application number:
**PCT/JP2024/011542**

(87) International publication number:
**WO 2024/203979 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.03.2023 JP 2023051708**

(71) Applicant: **NIPPON STEEL CHEMICAL &
MATERIAL CO., LTD.
Tokyo 103-0027 (JP)**

(72) Inventors:
• **SHINTA Ryuzo
Tokyo 103-0027 (JP)**
• **MATSUMURA Yasufumi
Tokyo 103-0027 (JP)**
• **ENOMOTO Yasushi
Tokyo 103-0027 (JP)**

(74) Representative: **Becker, Eberhard
Becker Kurig & Partner
Patentanwälte mbB
Bavariastraße 7
80336 München (DE)**

(54) **METAL-RESIN COMPOSITE AND USE FOR SAME**

(57) Provided are: a metal-resin composite comprising resin particles and a plurality of metal particles immobilized to the resin particles, wherein in the pH range of 3-6, the maximum value of zeta potential is not less than 5 mV, in the pH range of 8-10, the minimum value of the zeta potential is not more than -20 mV, and the zero charge point of the zeta potential exists in the pH range of 6.1-9.0; a labeling substance comprising the metal-resin composite; and an immunoassay method and an immunoassay reagent which use the labeling substance. The metal particles are particles of gold or an alloy thereof, and a CV value of the particle diameter of the metal particles is not more than 0.45.

FIG. 2A

**EP 4 671 763 A1**

## Description

Technical Field

The invention relates to a metal-resin composite that may be preferably used for applications such as immunological assays, a labeling substance using the same, an immunological assay method, an immunological assay reagent, an analyte measurement method, an analyte measurement kit, and a test strip for lateral flow chromatography.

Related Art

Immunoassay is a method for qualitatively and quantitatively analyzing trace components by utilizing specific reactions between antigens and antibodies. In immunoassays, detection sensitivity is increased by binding a labeling substance to antibodies, antigens, or composites thereof. Therefore, the labeling capability of the labeling substance may be said to be an important factor that affects the detection capability in immunoassays. It has been proposed to use composite particles in which metal particles and resin particles are composited as labeling substances used in immunoassays (Patent Documents 1 to 3).

Patent Document 1 discloses colored latex composed of gold nanoparticles bound to the surface of polymer-based latex particles. The colored latex of Patent Document 1 is obtained by irradiating gamma rays to a dispersion of styrene-acrylic acid copolymer latex and HAuCl, which is a precursor of gold nanoparticles, thereby binding gold nanoparticles to the surface of the latex. However, in the colored latex, since gold nanoparticles are bound only to the surface of the latex, there is a limitation on the loading amount of gold nanoparticles for expressing surface plasmon resonance, and the gold nanoparticles are easily detached. As a result, there is a concern that visibility and sensitivity as an immunological assay reagent may not be sufficient. In addition, since electromagnetic radiation such as gamma rays is irradiated, there is a concern of causing damage to the latex.

Patent Document 2 proposes a metal-resin composite in which a specific proportion of metal particles are present in the surface layer part of resin particles, which is excellent in durability and visibility, and enables highly sensitive determination in immunological assay without requiring addition of special equipment or work steps.

Patent Document 3 proposes a metal-resin composite that has excellent dispersibility in a state bound to many types of ligands such as antigens and antibodies, and blocking agents, and is less likely to cause aggregation, characterized by having a maximum value of zeta potential of 5 mV or more and a minimum value of -5 mV or less in the range of pH 3 to pH 10.

Non-Patent Document 1 discloses microgels in which gold nanoparticles are supported on poly-2-vinylpyridine latex particles, and confirms the pH responsiveness of the particle diameter of the microgels from changes in the behavior of localized surface plasmon resonance of the gold nanoparticles. However, in the microgels, gold nanoparticles are supported in a single layer near the surface layer of the latex particles. Therefore, the loading amount of gold nanoparticles is small, and it is considered that a deep color tone effective for immunoassays cannot be obtained. In addition, no examination has been conducted on the material, structure, composition, etc. of the microgels, and the effects on specific applications such as immunological assay reagent are unclear.

Citation List

Patent Literature

Patent Document 1: Japanese Patent Application Laid-Open Publication No. 2009-168495
Patent Document 2: International Publication WO2016/002742
Patent Document 3: International Publication WO2018/123952
Non-Patent Document 1: K.Akamatsu, M.Shimada, T.Tsuruoka, H.Nawafune, S.Fujii and Y.Nakamura; Langmuir 2010, 26, 1254-1259.

SUMMARY OF INVENTION

Technical Problem

In order to use metal-resin composites as labeling substances in immunological assay, it is necessary to stably bind them to ligands such as antigens and antibodies, and blocking agents. However, in the case of labeling ligands with metal-resin composites, even if a stable binding state may be formed, excellent detection sensitivity is not necessarily obtained. For example, fine metal-resin composites are prone to aggregation. In the case of aggregation, not only does handling performance significantly decrease, but unevenness in the concentration of the metal-resin composite as a labeling substance may occur, resulting in a significant decrease in detection sensitivity.

In addition, antigens, antibodies, and blocking agents used in immunological assay include various types such as proteins, synthetic polymers, and low molecular weight compounds, and there are those with strong anionic properties (for example, casein) and those with strong cationic properties (for example, whey). Therefore, if there were labeling substances that could use all of these antigens and antibodies, it would be very preferable for reasons such as material cost reduction.

The metal-resin composite of Patent Document 3 has improved dispersibility in a state bound to multiple types of ligands and blocking agents due to characteristic zeta potential behavior, and has high utility. Among the metal-resin composites of Patent Document 3, regarding gold-resin composites having gold particles and resin, those in which resin particles are treated with sodium hydroxide, which is a strong alkali (Example 4), and those treated with mercaptopropionic acid or polyglutamic acid after forming the gold-resin composite (Examples 5 and 7) have been manufactured. Through these treatments, the gold-resin composite exhibits characteristic zeta potential similar to platinum-resin composites having platinum particles and resin. However, the gold-resin composite manufactured in this way has low dispersibility in water, and in the case of application to immunochromatography as a labeling substance, problems such as clogging and color development in the background are observed, leaving room for improvement.

The object of the invention is to provide a metal-resin composite that takes advantage of the merits of metal-resin composites while having improved dispersibility in water and being able to avoid problems such as clogging in the case of application to immunochromatography as a labeling substance.

Solution to Problem

As a result of intensive research, the present inventors found that in a metal-resin composite in which multiple gold particles are immobilized on resin particles, the above problems may be solved by selecting the resin type used for the resin particles and controlling the particle diameter of the gold particles, and completed the invention.

That is, the metal-resin composite of the invention is a metal-resin composite having resin particles and multiple metal particles immobilized on the resin particles, in which

in the range of pH 3 to pH 6, the maximum value of zeta potential is 5 mV or more, and
in the range of pH 8 to pH 10, the minimum value of zeta potential is -20 mV or less, and
the zero charge point of zeta potential exists in the range of pH 6.1 to pH 9.0, and
the metal particles are particles of gold or an alloy thereof, and the Coefficient of Variation (CV value) of the particle diameter thereof is 0.45 or less.

In the metal-resin composite of the invention, the resin particles may include a first polymer and a second polymer of a different type from the first polymer. One of the first polymer and the second polymer may be a polymer having a cationic functional group, and the other may be a polymer having an anionic functional group.

In the metal-resin composite of the invention, the first polymer may be a polymer having a cationic functional group, and the second polymer may be a polymer having an anionic functional group. In this case, the molar ratio of the cationic functional group included in the first polymer and the anionic functional group included in the second polymer may be in the range of 99.5:0.5 to 80:20 as cationic functional group included in the first polymer:anionic functional group included in the second polymer.

Further, in the metal-resin composite of the invention, the first polymer may be a polymer having a substituent capable of adsorbing metal ions in its structure.

In the metal-resin composite of the invention, the average particle diameter of the metal particles may be in the range of 1 nm to 100 nm.

In the metal-resin composite of the invention, the metal particles may be formed by reducing metal ions adsorbed to the resin particles with a reducing agent solution in which a buffer solution adjusted to pH 3 to 8 and a reducing agent are mixed.

In the metal-resin composite of the invention, the average particle diameter thereof may be in the range of 30 nm to 1000 nm.

A labeling substance of the invention includes the above metal-resin composite. In this case, an antigen or antibody may be adsorbed to the surface of the metal-resin composite for use.

The immunological assay method of the invention uses the above labeling substance.

An immunological assay reagent of the invention includes the above labeling substance.

An analyte measurement method of the invention is a method for detecting or quantifying an analyte contained in a sample. This analyte measurement method uses a test strip for lateral flow chromatography that includes a membrane and a determination part in which a capture ligand that specifically binds to the analyte is immobilized on the membrane, and includes the following steps (I) to (III) including:

step (I): a step of bringing the analyte contained in the sample into contact with a labeled antibody in which an antibody that specifically binds to the analyte is labeled with a metal-resin composite;
step (II): a step of bringing the composite including the analyte and the labeled antibody formed in step (I) into contact with the capture ligand at the determination part; and
step (III): a step of measuring color intensity derived from light energy absorption due to localized surface plasmon resonance and/or electron transition of the metal-resin composite.

The metal-resin composite has resin particles and multiple metal particles immobilized on the resin particles, a maximum value of zeta potential is 5 mV or more in the range of pH 3 to pH 6, a minimum value of zeta potential is -20 mV or less in the range of pH 8 to pH 10, a zero charge point of zeta potential exists in the range of pH 6.1 to pH 9.0, the metal particles are particles of gold or an alloy thereof, and the CV value (Coefficient of Variation) of the particle diameter thereof is 0.45 or less.

An analyte measurement kit of the invention is an analyte measurement kit for detecting or quantifying an analyte contained in a sample. This analyte measurement kit includes a test strip for lateral flow chromatography that includes a membrane and a determination part in which a capture ligand that specifically binds to the analyte is immobilized on the membrane, and a detection reagent that includes a labeled antibody in which an antibody that specifically binds to the analyte is labeled with a metal-resin composite,

The metal-resin composite has a structure in which multiple metal particles are immobilized on resin particles, a maximum value of zeta potential is 5 mV or more in the range of pH 3 to pH 6, a minimum value of zeta potential is -20 mV or less in the range of pH 8 to pH 10, a zero charge point of zeta potential exists in the range of pH 6.1 to pH 9.0, the metal particles are particles of gold or an alloy thereof, and the CV value (Coefficient of Variation) of the particle diameter thereof is 0.45 or less.

A test strip for lateral flow chromatography of the invention is for detecting or quantifying an analyte contained in a sample. The test strip for lateral flow chromatography includes a membrane, a determination part in which a capture ligand that specifically binds to the analyte is immobilized on the membrane in the direction in which the sample develops, and a reaction part that is located upstream side of the determination part and includes a labeled antibody in which an antibody that specifically binds to the analyte is labeled with a metal-resin composite.

The metal-resin composite has a structure in which multiple metal particles are immobilized on resin particles, a maximum value of zeta potential is 5 mV or more in the range of pH 3 to pH 6, a minimum value of zeta potential is -20 mV or less in the range of pH 8 to pH 10, a zero charge point of zeta potential exists in the range of pH 6.1 to pH 9.0, the metal particles are particles of gold or an alloy thereof, and the CV value (Coefficient of Variation) of the particle diameter thereof is 0.45 or less.

Effects of Invention

The metal-resin composite of the invention exhibits characteristic zeta potential behavior through selection of the resin type used for the resin particles and control of the particle diameter of the gold particles. Therefore, while taking advantage of the strengths of conventional metal-resin composites, dispersibility in water is improved, and furthermore, defects such as clogging and background coloration are reduced in the case of application to immunochromatography as a labeling substance. Here, "zeta potential" means the potential of the slip plane of the electric double layer formed around the metal-resin composite in an acidic solution or alkaline solution, and is measured, for example, by electrophoretic light scattering method as the potential difference between an electrically neutral region sufficiently distant from the metal-resin composite and the slip plane.

In addition, the metal-resin composite of the invention may interact with various substances regardless of whether they are anionic or cationic due to its specific zeta potential, and therefore exhibits excellent dispersibility even in a state bound to many types of ligands such as antigens and antibodies, or to blocking agents, and aggregation is less likely to occur.

In addition, the metal-resin composite of the invention has a structure in which multiple metal particles are immobilized on resin particles, and therefore has a high loading amount of metal particles, and the metal particles are less likely to detach from the resin particles.

Furthermore, the metal particles exhibit light energy absorption due to electron transition in addition to localized surface plasmon resonance.

Therefore, the metal-resin composite of the invention is excellent in handling properties, durability, visibility, visual determination properties, and detection sensitivity. Accordingly, it may be preferably applied for purposes such as labeling substances in various immunological assays, immunological assay reagents, pharmaceuticals, solid catalysts, pigments, paints, conductive materials, electrodes, and sensor elements. In particular, in the case of using the metal-resin composite of the invention for immunological assay, it is excellent in handling properties, durability, and visibility, non-specific reactions are suppressed, and high-sensitivity determination becomes possible for various antigens and antibodies without requiring the addition of special equipment or work steps.

BRIEF DESCRIPTION OF DRAWINGS

[FIG. 1] A schematic diagram showing the cross-sectional structure of a metal-resin composite according to one embodiment of the invention.
[FIG. 2A] A schematic diagram showing the cross-sectional structure of one aspect of a metal-resin composite.
[FIG. 2B] A schematic diagram showing the cross-sectional structure of another aspect of a metal-resin composite.
[FIG. 3] An explanatory diagram showing an overview of an analyte measurement method using a test strip for lateral flow chromatography according to one embodiment of the invention.
[FIG. 4] A cross-sectional diagram of an immunochromatographic strip prepared in the Examples.

DESCRIPTION OF THE EMBODIMENTS

Hereinafter, embodiments of the invention will be described in detail with reference to the drawings as appropriate. The metal-resin composite of the present embodiment is applied to a labeling substance for immunological assay (hereinafter also simply referred to as "labeling substance") or an immunological assay reagent (hereinafter also simply referred to as "reagent").

[Metal-Resin Composite]

The metal-resin composite of the present embodiment includes resin particles and multiple metal particles immobilized on the resin particles, has a maximum value of zeta potential of 5 mV or more in the range of pH 3 to pH 6, has a minimum value of zeta potential of -20 mV or less in the range of pH 8 to pH 10, has a zero charge point of zeta potential in the range of pH 6.1 to pH 9.0, in which the metal particles are particles of gold or an alloy thereof, and the CV value (Coefficient of Variation) of the particle diameter is 0.45 or less.

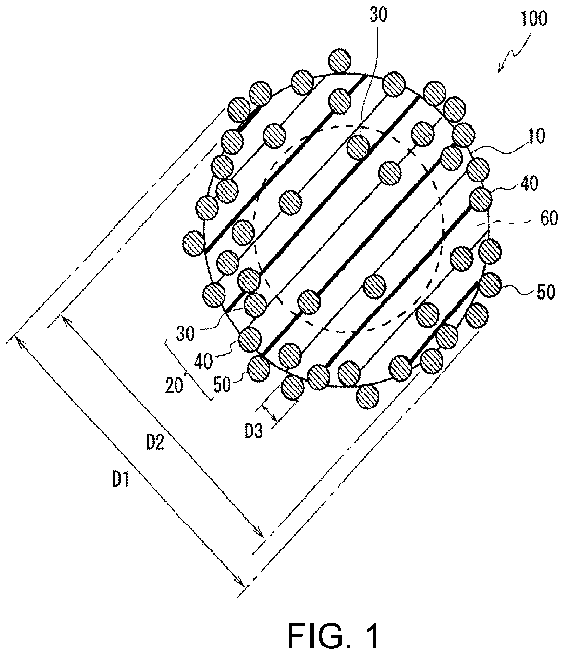

FIG. 1 is a cross-sectional schematic diagram of a metal-resin composite according to one embodiment of the invention. A metal-resin composite 100 includes resin particles 10 and metal particles 20. In the metal-resin composite 100, the metal particles 20 are immobilized on the resin particles 10. The resin particles 10 are preferably relatively larger particles than the metal particles 20. That is, in the metal-resin composite 100, multiple relatively small metal particles 20 are immobilized on large resin particles 10. In this case, as shown in FIG. 1, the relationship between the particle diameter D1 of the entire metal-resin composite 100, the particle diameter D2 of the resin particles 10, and the particle diameter D3 of the metal particles 20 is D1>D2>D3.
The average particle diameter of the metal-resin composite 100 (that is, the average of the particle diameter D1 in FIG. 1) is preferably, for example, 30 to 1000 nm. In the case of the average particle diameter of the metal-resin composite 100 being less than 30 nm, for example, the loading amount of the metal particles 20 tends to decrease, so coloration tends to be weaker than that of the metal particles 20 of the same size, and in the case of exceeding 1000 nm, there is a tendency for clogging to occur easily in the pores of chromatographic media such as membrane filters and for dispersibility to decrease according to use as a labeling substance or reagent. The average particle diameter of the metal-resin composite 100 has a preferable upper limit of 600 nm, more preferably 500 nm, from the viewpoint of improving dispersibility according to use as a labeling substance or reagent and obtaining high detection sensitivity in the case of using the metal-resin composite 100 for immunological assay. On the other hand, the preferable lower limit is 100 nm, more preferably 200 nm, and even more preferably 300 nm. In particular, in the case of the average particle diameter of the metal-resin composite 100 being 300 nm or more, excellent detection sensitivity may be stably obtained according to using the metal-resin composite 100 as a labeling substance for immunochromatography. Here, the particle diameter of the metal-resin composite 100 means a value obtained by adding the length of the protruding portion of partially exposed particles 40 or surface adsorbed particles 50 described later to the particle diameter of the resin particles 10, and may be measured by laser diffraction/scattering method, dynamic light scattering method, or centrifugal sedimentation method.

<Zeta Potential>

The metal-resin composite 100 has a maximum value of zeta potential of 5 mV or more in the range of pH 3 to pH 6, and has a minimum value of zeta potential value of -20 mV or less in the range of pH 8 to pH 10 (first characteristic). In the case of the zeta potential being in such a range, under acidic environment (for example, in the case of being dispersed in a dispersion medium having a pH of less than 7), the metal-resin composite 100 has cationic functional groups on the composite surface and carries a positive charge. On the other hand, under basic environment (for example, in the case of being dispersed in a dispersion medium having a pH exceeding 7), the metal-resin composite 100 has anionic functional groups on the composite surface and carries a negative charge. That is, in the case of binding an antigen, antibody, or blocking agent to its surface, the metal-resin composite 100 achieves a surface charge state suitable for binding of the antigen, antibody, or blocking agent corresponding to the pH of the dispersion medium (binding buffer). Specifically, under acidic environment, it may bind with strongly anionic antigens or antibodies, and under basic environment, it may bind with strongly cationic antigens or antibodies. That is, the metal-resin composite 100 may stably bind with any antigen or antibody regardless of being anionic or cationic. Therefore, it has high durability, stability, and versatility as a labeling substance for immunological assay and an immunological assay reagent.

On the other hand, in the case of the maximum value of zeta potential being less than 5 mV in the range of pH 3 to pH 6, or the minimum value of zeta potential value exceeding -20 mV in the range of pH 8 to pH 10, the metal-resin composite 100 has poor dispersibility due to electrostatic repulsion, and the metal-resin composite 100 itself tends to aggregate easily. Therefore, it becomes difficult to handle as a labeling substance for immunological assay and an immunological assay reagent, which is disadvantageous.

From such a viewpoint, it is preferable that the maximum value of the zeta potential is large, and it is preferable that the minimum value is small. In the range of pH 3 to pH 6, the maximum value of the zeta potential is preferably 10 mV or more, and more preferably 20 mV or more. Also, in the range of pH 8 to pH 10, the minimum value of the zeta potential is preferably -25 mV or less, and more preferably -30 mV or less.

Also, for the same reason, it is preferable that the metal-resin composite 100 has a difference between the maximum value and minimum value of the zeta potential of 20 mV or more in the range of pH 3 to pH 10 (second characteristic). More preferably, it is 40 mV or more, and even more preferably 60 mV or more.

Note that with the alkali treatment of the resin particles 10 disclosed in Patent Document 3, it was difficult to make the minimum value of the zeta potential of the gold-resin composite -20 mV or less in the range of pH 8 to pH 10. Also, with the mercaptopropionic acid treatment and polyglutamic acid treatment disclosed in Patent Document 3, although control of the zeta potential of the gold-resin composite is possible, there was a problem that the dispersibility in water is low and aggregation and precipitation tend to occur easily. In the present embodiment, as described later, the above problem is solved by selecting the resin type constituting the resin particles 10.

Also, the metal-resin composite 100 has a zero charge point of the zeta potential existing in the range of pH 6.1 to pH 9.0 (third characteristic). Here, the zero charge point is a point where the zeta potential is plus-minus zero. In the case of the zero charge point being within the above range, under weakly acidic to weakly basic environments where molecular biochemical reactions are normally performed, it is possible to make the surface charge of the metal-resin composite 100 into a wide range of charge states of both positive and negative polarities, which is preferable. In particular, in the case of satisfying the third characteristic in addition to the first characteristic and second characteristic, it means that the zeta potential changes greatly from the positive side to the negative side or from the negative side to the positive side in the range of pH 6.1 to pH 9.0, so excellent dispersibility may be exhibited in a wide pH range of both acidic environments including weakly acidic and basic environments including weakly basic. The preferable lower limit is pH 6.2, and more preferably pH 6.3. On the other hand, the preferable upper limit is pH 8.5, and more preferably pH 8.0. An even more preferable upper limit is pH 7.8.

On the other hand, in the case of the zero charge point being outside the above range, there is a tendency that it cannot be made into charge states of both positive and negative polarities unless under relatively strongly acidic or strongly basic environments.

In the metal-resin composite 100 of the present embodiment, the reason why the characteristic behavior of the zeta potential is exhibited is that the surface of the metal-resin composite particles 100 has both "cationic functional groups" and "anionic functional groups". This point will be described later.

<Resin Particles>

The resin particles 10 are not limited in their structure and composition, but are preferably particles of a polymer having substituents in the structure that is capable of adsorbing metal ions. Examples of polymers having substituents in the structure that is capable of adsorbing metal ions include polymers capable of adsorbing anionic ions and polymers capable of adsorbing cationic ions.

As polymers capable of adsorbing anionic ions, nitrogen polymers are particularly preferable. Nitrogen polymers are resins having nitrogen atoms in the main chain or side chain, and examples include polyamine, polyamide, polypeptide, polyurethane, polyurea, polyimide, polyimidazole, polyoxazole, polypyrrole, polyaniline, and the like. Preferably, they are polyamines such as poly-2-vinylpyridine, poly-3-vinylpyridine, poly-4-vinylpyridine, and the like. Moreover, in the case of having nitrogen atoms in the side chain, for example, acrylic resins, phenol resins, epoxy resins, and the like may be widely used. The nitrogen atoms in the nitrogen polymer easily chemisorb anionic ions such as [AuCl4]- as precursors of gold particles, which are the metal particles 20 that have excellent visibility and facilitate immobilization of antigens or antibodies. In the present embodiment, since the metal ions adsorbed in the nitrogen polymer are reduced to form the metal particles 20, part of the generated metal particles 20 become the inclusion particles 30 or the partially exposed particles 40. Note that anionic ions of metal compounds such as platinum, palladium, silver, nickel, copper, and the like may also be used similarly.

Moreover, polymers capable of adsorbing cationic ions are resins having carboxylic acid groups, sulfonic acid groups, and the like in the main chain or side chain. Specific examples of polymers capable of adsorbing cationic ions include, for example, carboxylic acid group-containing polymers such as acrylic acid polymers and sulfonic acid group-containing polymers such as polystyrene sulfonic acid, and more specifically, polyacrylic acid, vinyl carboxylic acid, polyvinyl acetate, polyvinyl sulfonic acid, polystyrene sulfonic acid, and the like may be mentioned. Polymers capable of adsorbing cationic ions may chemisorb cationic ions such as Au+ by the carboxylic acid groups, sulfonic acid groups, and the like contained therein. For example, by reducing the chemisorbed Au+ to form the metal particles 20 (in this case, gold particles), it is possible to produce a structure similar to the above nitrogen polymer particles. Note that other cationic ions such as platinum, palladium, silver, nickel, copper, and the like may also be used similarly.

Since polymers capable of adsorbing anionic ions are cationic, they exhibit a function of making the zeta potential of the metal-resin composite 100 positive under acidic environments. On the other hand, since polymers capable of adsorbing cationic ions are anionic, they exhibit a function of making the zeta potential of the metal-resin composite 100 negative under basic environments.

Note that polymers other than polymers having substituents capable of adsorbing metal ions in their structure include, for example, polystyrene and the like, but in this case, it is difficult to adsorb metal ions inside the resin particles 10. As a result, most of the generated the metal particles 20 become the surface adsorbed particles 50. As described later, since the surface adsorbed particles 50 have a small contact area with the resin particles 10, the adhesive force between the resin particles 10 and the metal is small, and the metal particles 20 tend to easily detach from the resin particles 10.

The polymer having substituents capable of adsorbing metal ions in its structure may be a copolymer with known polymerizable monomers. Here, examples of the copolymer include random copolymers, block copolymers, alternating copolymers, and crosslinked polymers. Additionally, two or more types of monomers may be copolymerized to form the resin particles 10, or monomers may be reacted with functional groups present on the surface of the resin particles 10 and further polymerized using them as polymerization active terminals. The copolymerization composition is not limited, but it is preferable that the monomer containing substituents capable of adsorbing the metal ions is 10 mol% or more. The polymerizable monomer may be selected without restriction according to the application of the metal-resin composite 100. For example, for applications of improving the shape, size uniformity, and dispersion stability of the resin particles 10, polymerizable monomers having characteristics as surfactants may be used. Examples of such polymerizable monomers include polyethylene glycol methyl ether methacrylate and polyethylene glycol dimethacrylate.

Additionally, the resin particles 10 of the present embodiment preferably include two or more types of polymers. The metal-resin composite 100 has both "cationic functional groups" and "anionic functional groups" on its surface, whereby the first to third characteristics, which are characteristic behaviors of zeta potential, are effectively expressed. Therefore, in the case where the resin particles 10 include a first polymer and a second polymer of a different type from the first polymer, it is preferable that either the first polymer or the second polymer is a "polymer having cationic functional groups" and the other is a "polymer having anionic functional groups. "Here, examples of "cationic functional groups" include nitrogen-containing groups such as pyridinyl groups, amino groups, amide groups, imide groups, and imidazole groups. Additionally, examples of "anionic functional groups" include carboxyl groups, sulfonic acid groups, phosphonic acid groups, phosphoric acid groups, silanol groups, and the like.

Additionally, the resin particles 10 of the present embodiment may be a copolymer having polymer segments having the cationic functional groups and polymer segments having the anionic functional groups. Here, the preferable cationic functional groups and anionic functional groups are as described above. Additionally, the copolymerization form of the copolymer may take forms such as random copolymers, block copolymers, alternating copolymers, and the like.

For example, in the case where the first polymer is polyvinylpyridine, which is a nitrogen polymer, and the second polymer is polymethacrylic acid, the pyridine contained in the polyvinylpyridine is a "cationic functional group," and the carboxyl group contained in the polymethacrylic acid is an "anionic functional group." In an acidic environment, the nitrogen of pyridine becomes positively charged in the NH+ state, and the carboxyl group becomes uncharged in the COOH state, so the metal-resin composite 100 has an overall positive surface charge. On the other hand, in a basic environment, the nitrogen of pyridine becomes uncharged in the N state, and the carboxyl group becomes negatively charged in the COO- state, so the metal-resin composite 100 has an overall negative surface charge. As a result, it is considered that the zeta potential of the metal-resin composite 100 exhibits the above-described first to third characteristics.

On the other hand, in Patent Document 3, regarding gold-resin composites, carboxyl groups, which are "anionic functional groups," were generated in polyvinylpyridine, which is a nitrogen polymer, by hydrolysis of the surface of the resin particles 10 through strong alkali treatment, or carboxyl groups were introduced into the metal-resin composite 100 through mercaptopropionic acid treatment or polyglutamic acid treatment. However, hydrolysis of the surface of the resin particles 10 causes damage to the resin particles 10 themselves due to strong alkali, which may have resulted in large variations in the particle diameter of gold particles. Additionally, in mercaptopropionic acid treatment and polyglutamic acid treatment, molecules containing carboxyl groups were merely adsorbed on the surface of the gold-resin composite, making them prone to desorption, which is considered to have led to decreased dispersibility in water. In contrast, in the present embodiment, the resin particles 10 include the second polymer polymerized on the surface or inside of the first polymer, whereby the "anionic functional groups" do not desorb from the resin particles 10, and the function as "anionic functional groups" is effectively maintained. This is considered to be the reason why the above-described first to third characteristics are sufficiently exhibited, dispersibility in water is enhanced, and a stable dispersed state may be maintained.

In general, polymers capable of adsorbing anionic ions often have "cationic functional groups," and polymers capable of adsorbing cationic ions often have "anionic functional groups. "Therefore, as a combination of the first polymer and the second polymer, the first polymer may be selected from polymers capable of adsorbing anionic ions and the second polymer may be selected from polymers capable of adsorbing cationic ions, or the first polymer may be selected from polymers capable of adsorbing cationic ions and the second polymer may be selected from polymers capable of adsorbing anionic ions.

In the case where the first polymer is the main polymer constituting the resin particles 10, it is presumed to be preferable that the second polymer exists on the surface or inside of the first polymer at the surface of the resin particles 10. Therefore, the molar ratio of cationic functional groups contained in the first polymer to anionic functional groups contained in the second polymer (cationic functional groups contained in the first polymer : anionic functional groups contained in the second polymer) is preferably within a range of, for example, 99.5:0.5 to 80:20. Regarding the molar ratio of cationic functional groups contained in the first polymer, a more preferable upper limit is 99, and a more preferable lower limit is 90. According to the ratio of cationic functional groups contained in the first polymer to anionic functional groups contained in the second polymer being within the above range, the "cationic functional groups" and "anionic functional groups" become optimal amounts, and control of zeta potential becomes easier.

<Metal Particle>

In the metal-resin composite 100, the metal particle 20 is a particle of gold or an alloy thereof. A gold-resin composite using gold particles as the metal particle 20 is less likely to cause aggregation in a state bound with ligands such as antibodies compared to metal-resin composites having particles of other metal species, and has extremely excellent dispersibility. Furthermore, it has excellent visibility and easy immobilization of antigens or antibodies. In particular, by controlling the particle diameter of gold particles and the inter-particle distance between gold particles, various colors such as red, purple, and blue are exhibited. Therefore, in the case of using a gold-resin composite as a labeling substance for immunochromatography, labeling substances of various colors may be obtained. Moreover, in the case where the metal particle 20 is a particle of gold or an alloy thereof, the effects of the invention are more greatly exhibited. Here, the gold alloy means an alloy composed of gold and metal species other than gold, containing gold in an amount of 1% by weight or more, preferably 10% by weight or more, more preferably 50% by weight or more, and further preferably 60% by weight or more. In this case, other metal species forming an alloy with gold are not particularly limited, but for example, platinum, silver, nickel, copper, palladium, and the like are preferable.

Moreover, the metal particle 20 may have particles of other metals together with particles of gold or an alloy thereof. As such metals, platinum, palladium, silver, nickel, and copper are preferable from the viewpoint of ease of nano-size composite formation of metal and resin. These metals may be used as simple substances or alloys. For example, a platinum alloy means an alloy composed of platinum and metal species other than platinum, containing platinum in an amount of 1% by weight or more, preferably 10% by weight or more, more preferably 50% by weight or more, and further preferably 60% by weight or more.

Note that even in the case where the metal particle 20 is a particle of a metal other than gold or an alloy thereof, it is possible to control the zeta potential in the same manner as the invention.

Moreover, the average particle diameter of gold particles as the metal particle 20 measured by scanning electron microscope (SEM) observation (that is, the average of particle diameter D3 in FIG. 1) is preferably, for example, 1 to 100 nm. In the case where the average particle diameter of gold particles is less than 1 nm or exceeds 100 nm, localized surface plasmon resonance and light energy absorption by electron transition are less likely to be exhibited, so sensitivity tends to decrease.

Moreover, the average particle diameter of gold particles has a preferable upper limit of 25 nm, more preferably 20 nm, and further preferably 15 nm from the viewpoint of obtaining high detection sensitivity as a labeling substance for immunological assay and an immunological assay reagent. On the other hand, the preferable lower limit is 2 nm. In the metal-resin composite 100 of the present embodiment, the average particle diameter of gold particles may be reduced to 20 nm or less by using a specific reducing agent solution described later in the step of reducing gold ions to gold particles.

Moreover, the CV value (Coefficient of Variation) of the particle diameter of gold particles as the metal particle 20 is 0.45 or less. In the case where the CV value exceeds 0.45, variation in results tends to increase according to application to immunochromatography, and the reliability of immunological testing decreases. Therefore, the CV value is preferably 0.44 or less, and more preferably 0.43 or less. In the metal-resin composite 100 of the present embodiment, the CV value of the particle diameter of gold particles may be controlled to 0.45 or less by using a specific reducing agent solution in the step of reducing gold ions to gold particles.

<Existence State of Metal Particles>

The existence state of the metal particle 20 in the metal-resin composite 100 is not limited. For example, the metal particle 20 may be two-dimensionally distributed on the surface of the resin particles 10, or the metal particle 20 may be included inside the resin particles 10. Moreover, a core-shell structure may be formed with the resin particles 10 as a shell and the metal particle 20 as a core.

Moreover, a part of the metal particle 20 may be three-dimensionally distributed in a surface layer part 60 of the resin particles 10. In this case, a part of the three-dimensionally distributed metal particle 20 may be partially exposed outside the resin particles 10, and the remaining part may be included in the resin particles 10. Specifically, as shown in FIG. 1, it is preferable that the metal particle 20 includes metal particles completely included in the resin particles 10 (also referred to as "inclusion particle 30"), and metal particles having a portion embedded in the resin particles 10 and a portion exposed outside the resin particles 10 (also referred to as "partially exposed particles 40"). Moreover, in addition to this, metal particles adsorbed on the surface of the resin particles 10 (also referred to as "surface adsorbed particles 50") may be present.

In the case of using the metal-resin composite 100 for a labeling substance for immunological assay or an immunological assay reagent, an antigen, antibody, or blocking agent is immobilized on the surface of the resin particles 10 or the surface of the partially exposed particles 40 or the surface adsorbed particles 50 for use. At that time, the antigen, antibody, or blocking agent is immobilized on the partially exposed particles 40 and the surface adsorbed particles 50, while it is considered difficult to be immobilized on the inclusion particle 30. However, since all of the partially exposed particles 40, the surface adsorbed particles 50, and the inclusion particle 30 exhibit light energy absorption by electron transition in addition to localized surface plasmon resonance, not only the partially exposed particles 40 and the surface adsorbed particles 50, but also the inclusion particle 30 contributes to improving the visibility of the labeling substance for immunological assay and the immunological assay reagent. Furthermore, the partially exposed particles 40 and the inclusion particle 30 have a larger contact area with the resin particles 10 compared to the surface adsorbed particles 50, and in addition, an anchor effect due to the embedded state is achieved, so the physical adsorption force is strong and they are difficult to detach from the resin particles 10. Moreover, the antigen, antibody, or blocking agent adsorbed on the partially exposed particles 40 or the surface adsorbed particles 50 forms coordinate bonds with the metal, making them difficult to detach. Therefore, the durability and stability of the labeling substance for immunological assay and the immunological assay reagent using the metal-resin composite 100 may be made excellent.

In the metal-resin composite 100, the inclusion particle 30 has its entire surface covered by the resin constituting the resin particles 10. Moreover, the partially exposed particles 40 have 5% or more and less than 100% of their surface area covered by the resin constituting the resin particles 10, and from the viewpoint of durability of the labeling substance for immunological assay and the immunological assay reagent, the lower limit is preferably 20% or more of the surface area, and more preferably 30% or more. Furthermore, the surface adsorbed particles 50 preferably have more than 0% and less than 5% of their surface area covered by the resin constituting the resin particles 10.

Moreover, the loading amount of the metal particles 20 (total of the inclusion particles 30, the partially exposed particles 40, and the surface adsorbed particles 50) on the metal-resin composite 100 is preferably 3% by weight to 80% by weight relative to the weight of the metal-resin composite 100. Within this range, the metal-resin composite 100 excels in visibility, visual determination, and detection sensitivity as a labeling substance. In the case of the loading amount of the metal particles 20 being less than 3% by weight, the immobilization amount of antibody or antigen tends to decrease, and detection sensitivity tends to decline. On the other hand, in the case of the loading amount of the metal particles 20 exceeding 80% by weight, the particle diameter of the metal particles 20 increases significantly, and the light absorption characteristics by the metal particles 20 tend to decline. The loading amount of the metal particles 20 is more preferably 15% by weight to 70% by weight, and even more preferably 15% by weight to 60% by weight.

Moreover, it is preferable that 10% by weight to 90% by weight of the metal particles 20 are the partially exposed particles 40 and the surface adsorbed particles 50. Within this range, a sufficient immobilization amount of antibody or antigen on the metal particles 20 may be secured, resulting in high sensitivity as a labeling substance. It is more preferable that 20% by weight to 80% by weight of the metal particles 20 are the partially exposed particles 40 and the surface adsorbed particles 50, and from the viewpoint of durability of the labeling substance for immunological assay and the immunological assay reagent, it is even more preferable that the surface adsorbed particles 50 are 20% by weight or less.

Moreover, in the case of using the metal-resin composite 100 for immunological assay, in order to obtain excellent detection sensitivity, it is preferable that 60% by weight to 100% by weight, preferably 75% by weight to 100% by weight, more preferably 85% by weight to 100% by weight of the metal particles 20 are present in the surface layer part 60, and even more preferably present within a range of 40% of the particle radius in the depth direction from the surface of the resin particles 10. Moreover, it is preferable that 5% by weight to 90% by weight of the metal particles 20 present in the surface layer part 60 are the partially exposed particles 40 or the surface adsorbed particles 50, because a sufficient immobilization amount of antibody or antigen on the metal particles 20 may be secured, resulting in high sensitivity as a labeling substance. In other words, it is preferable that 10% by weight to 95% by weight of the metal particles 20 present in the surface layer part 60 are the inclusion particles 30.

Here, the "surface layer part" means a range from the surface of the resin particles 10 to 50% of the particle radius in the depth direction, based on the outermost position of the metal-resin composite 100 (that is, the protruding end portions of the partially exposed particles 40 or the surface adsorbed particles 50). Moreover, the "three-dimensional distribution" means that the metal particles 20 are dispersed not only in the surface direction of the resin particles 10 but also in the depth direction.

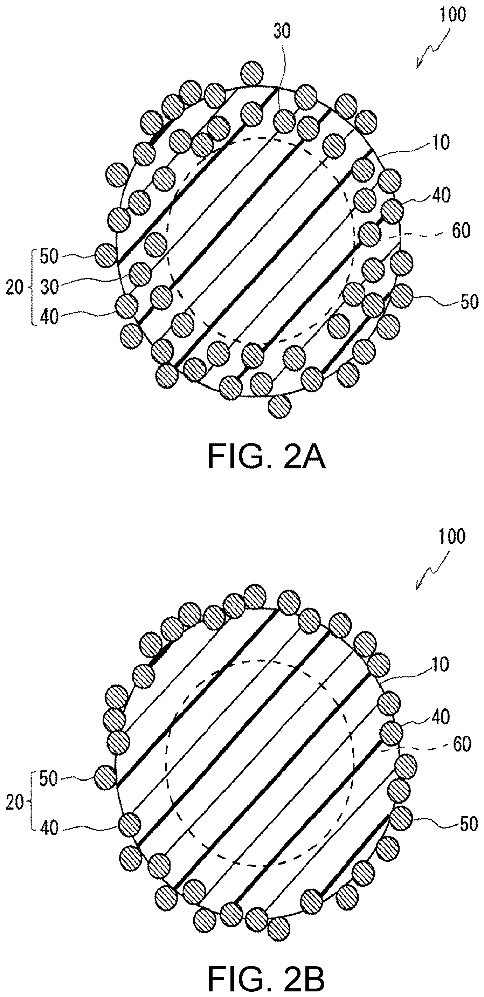

As described above, since the inclusion particles 30 also exhibit light energy absorption due to electron transition in addition to localized surface plasmon resonance, not only the partially exposed particles 40 and the surface adsorbed particles 50, but also the inclusion particles 30 contribute to improving the visibility of labeling substances for immunological assay and immunological assay reagent. From the viewpoint of such visibility improvement, it is preferable that in the metal-resin composite 100, for example as shown in FIG. 2A, the inclusion particles 30 are distributed concentrated within a certain range in the depth direction from the surface of the resin particles 10, and no inclusion particles 30 are present near the center of the resin particles 10. More specifically, in order to effectively exhibit light energy absorption due to electron transition in addition to localized surface plasmon resonance by the inclusion particles 30, for example, in the case of the particle diameter D2 of the resin particles 10 being 800 nm, it is preferable that 70% by weight or more, preferably 80% by weight or more, more preferably 90% to 100% by weight of the inclusion particles 30 are present within a range of, for example, 0 to 200 nm in the depth direction from the surface of the resin particles 10. In particular, in the case of the region where all (100% by weight) of the inclusion particles 30 are distributed (inclusion particle distribution region) being within a range of, for example, 0 to 100 nm from the surface of the resin particles 10, it is preferable because light energy absorption due to electron transition may be maximized in addition to localized surface plasmon resonance by the inclusion particles 30.

Moreover, the metal-resin composite 100 may not have the inclusion particles 30. For example, as shown in FIG. 2B, in the metal-resin composite 100, all of the metal particles 20 may be fixed to the surface of the resin particles 10 without overlapping in the radial direction of the resin particles 10. In this case, the metal particles 20 are composed of the partially exposed particles 40 and/or the surface adsorbed particles 50.

[Method for Manufacturing Metal-Resin Composite]

The method for manufacturing the metal-resin composite 100 is not particularly limited, but one example is as follows. Here, a case where the first polymer is polyvinylpyridine as a nitrogen polymer and the second polymer is polymethacrylic acid is taken as an example.

First, a dispersion of polyvinylpyridine particles is manufactured by emulsion polymerization. Next, an aqueous solution of methacrylic acid is added dropwise to the obtained dispersion to perform soap-free polymerization. As a result, polymethacrylic acid is incorporated into the polyvinylpyridine particles, and a dispersion of the resin particles 10 having multiple carboxyl groups derived from methacrylic acid on the surface or inside is obtained. At this time, it is presumed that on the surface or inside of the resin particles 10, a structure is formed in which polymethacrylic acid as the second polymer is present in polyvinylpyridine as the first polymer.

Next, a solution containing metal ions is added to the dispersion of the resin particles 10 to adsorb the metal ions to the resin particles 10 (hereinafter referred to as "metal ion adsorbed resin particles"). For example, in the case of generating gold particles as the metal particles 20, a chloroauric acid ($HAuCl_4$) aqueous solution or the like may be used as the solution containing gold ions. Moreover, a gold complex may be used instead of gold ions.

Moreover, as a solvent for the solution containing metal ions, instead of water, hydrous alcohol or alcohol such as methanol, ethanol, n-propanol, isopropanol, n-butanol, sec-butanol, t-butanol, or acids such as hydrochloric acid, sulfuric acid, nitric acid may be used.

Moreover, additives such as water-soluble polymer compounds like polyvinyl alcohol, surfactants, alcohols; ethers such as tetrahydrofuran, diethyl ether, diisopropyl ether; polyols such as alkylene glycol, polyalkylene glycol, monoalkyl ethers or dialkyl ethers thereof, glycerin; various water-miscible organic solvents such as ketones like acetone, methyl ethyl ketone may be added to the solution as necessary. Such additives are effective for promoting the reduction reaction rate of metal ions and controlling the size of the generated metal particles 20.

Next, by adding the metal ion adsorbed resin particles to a reducing agent solution, the metal ions may be reduced to generate the metal particles 20, and the metal-resin composite 100 may be obtained. Known reducing agents may be used. Examples of the reducing agent include sodium borohydride, dimethylamine borane, citric acid, sodium hypophosphite, hydrazine hydrate, hydrazine hydrochloride, hydrazine sulfate, formaldehyde, sucrose, glucose, ascorbic acid, erythorbic acid, sodium phosphinate, hydroquinone, Rochelle salt, and the like. Among these, sodium borohydride, dimethylamine borane, or citric acid is preferable.

As the reducing agent solution in the reduction step, a reducing agent solution prepared by mixing a buffer solution adjusted to preferably pH 3 to 8, more preferably pH 4 to 7, and even more preferably pH 5 to 6.5, with a reducing agent may be used. By using such a reducing agent solution, the average particle diameter of gold particles as the metal particles 20 may be easily controlled to 20 nm or less, and the CV value of the particle diameter may also be made 0.45 or less. The reason why it was difficult to control the average particle diameter of gold particles to 20 nm or less in the gold-resin composite of Patent Document 3 is considered to be that the pH of the reducing agent solution shifts to the acidic side due to the influence of hydrogen ions and halogen ions generated from metal halides such as chloroauric acid during the reduction process, and as a result, hydrogen ions and halogen ions become difficult to desorb from the resin particles 10, the reduction reaction becomes slow, and the gold particles enlarge. The reducing agent solution prepared by mixing a buffer solution with adjusted pH and a reducing agent promotes the action of halogen ions desorbing from the resin particles 10, thereby accelerating the reduction reaction and generating numerous nuclei of gold particles, and as a result, it is presumed that the average particle diameter of gold particles becomes easier to control to 20 nm or less.

Here, the compound used in the buffer solution is not particularly limited, but in order to prevent desorption of metal ions from the resin particles 10 and perform uniform nucleation and nuclear growth of gold particles, it is preferable to use a main buffering agent in combination with a basic compound that exhibits basicity of pH 7 or higher according to dissolution in water as an additive to the main buffering agent. As the main buffering agent, for example, a Good's buffering agent having a pKa in the range of 5.5 to 7.5 at 20°C may be used. Good's buffering agent that are preferably used include, for example, 2-morpholinoethanesulfonic acid (MES), bis(2-hydroxyethyl)iminotris(hydroxymethyl)methane (Bis-Tris), N-(2-acetamido)iminodiacetic acid (ADA), piperazine-1,4-bis(2-ethanesulfonic acid) (PIPES), N-(2-acetamido)-2-aminoethanesulfonic acid (ACES), and the like. Examples of basic compounds as additives include buffering agents having a pKa in the range of 7.6 to 12 at 20°C, alkali metal compounds, nitrogen-containing compounds, and the like, with nitrogen-containing compounds that exhibit basicity of pH 7 or higher according to dissolution in water being particularly preferable. Nitrogen-containing compounds preferably used in the reduction step include 2-amino-2-(hydroxymethyl)-1,3-propanediol, ammonia, methylamine, dimethylamine, trimethylamine, triethylamine, monoethanolamine, diethanolamine, triethanolamine methyl, dimethylaminoethanol, isopropylaminoethanol, isobutylamine, morpholine, and the like. In the case of using a main buffering agent and a basic compound in combination, for example, a molar ratio of 2:8 to 8:2 is preferable, and a molar ratio of 3:7 to 7:3 is more preferable. The buffer solution may have its pH adjusted by adding acids such as hydrochloric acid and sulfuric acid, or alkalis such as sodium hydroxide and potassium hydroxide as necessary. Furthermore, in the reduction step, it is preferable to use a surfactant in combination for the purpose of forming a stable metal-resin composite 100. Examples of surfactants include nonionic surfactants. Nonionic surfactants preferably used in the reduction step include polyethylene glycol octylphenyl ether (TritonX-100), polyoxyethylene sorbitan monolaurate (Tween20), glycerin laurate, glycerin monostearate, sorbitan fatty acid ester, polyoxyethylene alkyl ether, pentaethylene glycol monododecyl ether, polyoxyethylene alkylphenyl ether, octylphenol ethoxylate, nonylphenol ethoxylate, polyoxyethylene polyoxypropylene glycol, and the like.

Furthermore, the particle diameter of the generated metal particles 20 may also be controlled by adjusting the reduction rate of metal ions according to the temperature of the reducing agent solution.

In reducing the metal ions in the metal ion adsorbed resin particles to generate the metal particles 20, the metal ion adsorbed resin particles may be added to the reducing agent solution, or the reducing agent may be added to the metal ion adsorbed resin particles, but the former is preferable from the viewpoint of ease of generation of the inclusion particles 30 and the partially exposed particles 40.

Furthermore, in order to maintain the dispersibility of the metal-resin composite 100 in water, dispersants such as citric acid, poly-L-lysine, polyvinylpyrrolidone, polyvinylpyridine, polyvinyl alcohol, DISPERBYK194, DISPERBYK180, DISPERBYK184 (manufactured by BYK-Chemie Japan K.K.), etc. may be added.

Furthermore, the pH may be adjusted using buffering agents such as boric acid or phosphoric acid, acids such as hydrochloric acid or sulfuric acid, or alkalis such as sodium hydroxide or potassium hydroxide to maintain dispersibility.

The metal-resin composite 100 having the above configuration may be preferably applied as a labeling substance to various immunological assay methods, particularly by adsorbing antigens or antibodies to the surface of the metal particles 20. Furthermore, it may be preferably applied as a material for immunological assay labeling substances or immunological assay reagents that excel in visual determination performance particularly in low concentration regions (high sensitivity regions). In the present embodiment, as described above, the resin particles 10 include the second polymer polymerized on the surface or inside of the first polymer, thereby obtaining the resin particles 10 that achieve both anionic and cationic properties. As a result, the above first to third characteristics regarding zeta potential may be expressed, while enhancing dispersibility in water and maintaining a stable dispersed state.

Furthermore, there is no particular limitation on the form of the immunological assay labeling substance or immunological assay reagent, but for example, it may be used as a dispersion in which the metal-resin composite 100 is dispersed in water or a buffer solution with adjusted pH.

The method for adsorbing antigens or antibodies to the surface of the metal particles 20 is not particularly limited, and known methods using physical adsorption and chemisorption may be used. The method using chemisorption is preferable because the binding between the metal particles 20 and the antigens or antibodies becomes strong. Physical adsorption and chemisorption may be used in combination.

Examples of physical adsorption include a method of immersing the metal-resin composite 100 in a buffer solution containing antigens or antibodies and incubating, a method of immersing the metal-resin composite 100 in a buffer solution and further adding antigens or antibodies, and the like.

Examples of chemisorption include a method of introducing SH groups into antigens or antibodies and reacting them with the metal-resin composite 100 to form metal-SH bonds, a method of introducing carboxyl groups to the surface of the metal-resin composite 100, then succinimidylating and reacting with amino groups of antigens or antibodies to form chemical bonds, and the like. Compounds for introducing carboxyl groups to the surface of the metal-resin composite 100 are suitably compounds having both functional groups with coordination bonding properties to metals, such as amino groups, SH groups, carbonyl, amide, imide, and carboxyl groups. SH groups are preferable as functional groups because coordination bonds with metals are strong, and examples of compounds having SH groups and carboxyl groups at both terminals include mercaptopropionic acid, mercaptoundecanoic acid, mercaptolauric acid, and the like. Furthermore, polymer compounds having multiple functional groups with coordination bonding properties to metals and carboxyl groups within one molecule may easily obtain stable bonds with the metal-resin composite 100. For example, polyglutamic acid and polyaspartic acid, which are polypeptides of glutamic acid and aspartic acid, are more preferable as polymer compounds for introducing carboxyl groups to the surface of the metal-resin composite 100.

Next, an analyte measurement method using the metal-resin composite 100 as a labeling substance, a test strip for lateral flow chromatography, and an analyte detection/quantification kit will be described.

[Test Strip for Lateral Flow Chromatography]

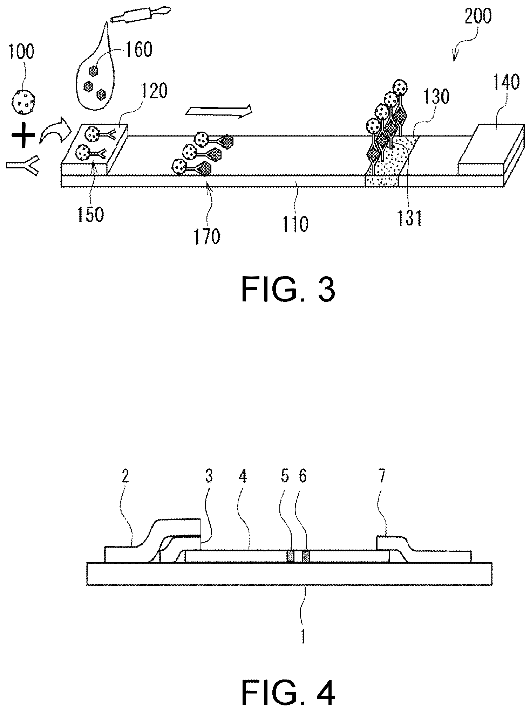

First, with reference to FIG. 3, a test strip for lateral flow chromatography (hereinafter sometimes simply referred to as "test strip") according to one embodiment of the invention will be described. A test strip 200 may be preferably used in the analyte measurement method according to one embodiment of the invention, as described later.

The test strip 200 includes a membrane 110. The membrane 110 is provided with a sample addition part 120, a determination part 130, and a liquid suction part 140 in this order in the sample development direction.

<Membrane>

As the membrane 110 in the test strip 200, those used as membrane materials in general test strips may be applied. The membrane 110 is formed of an inert substance (a substance that does not react with an analyte 160, various ligands, etc.) made of a fine porous material that exhibits capillary action and allows the sample to develop simultaneously with the addition of the sample. Specific examples of the membrane 110 include fibrous or non-woven fibrous matrices, membranes, filter papers, glass fiber filter papers, cloths, cotton, etc. composed of polyurethane, polyester, polyethylene, polyvinyl chloride, polyvinylidene fluoride, nylon, cellulose derivatives, and the like. Among these, membranes, filter papers, glass fiber filter papers, etc. composed of cellulose derivatives or nylon are preferably used, and more preferably nitrocellulose membranes, mixed nitrocellulose ester (mixture of nitrocellulose and cellulose acetate) membranes, nylon membranes, and filter papers are used.

The test strip 200 preferably includes a support that supports the membrane 110 in order to make the operation more convenient. As the support, for example, plastic or the like may be used.

<Sample Addition Part>

The test strip 200 may have a sample addition part 120 for adding a sample including the analyte 160. The sample addition part 120 is a site for receiving a sample including the analyte 160 in the test strip 200. The sample addition part 120 may be formed on the membrane 110 on the upstream side of the determination part 130 in the direction in which the sample develops, or alternatively, a sample addition pad composed of materials such as cellulose filter paper, glass fiber, polyurethane, polyacetate, cellulose acetate, nylon, cotton cloth, etc. may be provided on the membrane 110 to constitute the sample addition part 120.

<Determination Part>

A capture ligand 131 that specifically binds to the analyte 160 is immobilized in the determination part 130. The capture ligand 131 may be used without particular limitation as long as it forms a specific binding with the analyte 160, and for example, an antibody against the analyte 160 may be preferably used. The capture ligand 131 is immobilized so that it does not move from the determination part 130 even in the case of providing a sample to the test strip 200. The capture ligand 131 may be directly or indirectly immobilized to the membrane 110 by physical or chemical bonding, adsorption, or the like.

In addition, the determination part 130 is not particularly limited as long as it has a configuration such that a composite 170 including a labeled antibody 150 and the analyte 160 contacts the capture ligand 131 that specifically binds to the analyte 160. For example, the capture ligand 131 may be directly immobilized to the membrane 110, or alternatively, the capture ligand 131 may be immobilized to a pad made of cellulose filter paper, glass fiber, nonwoven fabric, or the like that is immobilized to the membrane 110.

<Liquid Suction Part>

The liquid suction part 140 is formed by, for example, a pad of water-absorbing material such as cellulose filter paper, nonwoven fabric, cloth, cellulose acetate, or the like. The movement speed of the sample after the development front (front line) of the added sample reaches the liquid suction part 140 varies depending on the material, size, and other factors of the liquid suction part 140. Therefore, by selecting the material, size, and other factors of the liquid suction part 140, an optimal speed for detection and quantification of the analyte 160 may be set. Note that the liquid suction part 140 is an optional configuration and may be omitted.

The test strip 200 may further include optional parts such as a reaction part, a control part, and the like, as necessary.

<Reaction Part>

Although not illustrated, the test strip 200 may have a reaction part including the labeled antibody 150 formed on the membrane 110. The reaction part may be provided upstream of the determination part 130 in the direction in which the sample flows. Note that the sample addition part 120 in FIG. 3 may be used as the reaction part. In the case of the test strip 200 having a reaction part, according to providing a sample including the analyte 160 to the reaction part or the sample addition part 120, the analyte 160 included in the sample and the labeled antibody 150 may be brought into contact in the reaction part. In this case, by simply providing the sample to the reaction part or the sample addition part 120, the composite 170 including the analyte 160 and the labeled antibody 150 may be formed, so that a so-called one-step type immunochromatography becomes possible.

The reaction part is not particularly limited as long as it includes the labeled antibody 150 that specifically binds to the analyte 160, but may be one in which the labeled antibody 150 is directly applied to the membrane 110. Alternatively, the reaction part may be one in which a pad (conjugate pad) made of, for example, cellulose filter paper, glass fiber, nonwoven fabric, or the like impregnated with the labeled antibody 150 is immobilized to the membrane 110.

<Control Part>

Although not illustrated, the test strip 200 may have a control part formed on the membrane 110 downstream of the determination part 130 in the direction in which the sample develops, where a capture ligand that specifically binds to the labeled antibody 150 is immobilized. By measuring the color intensity at both the determination part 130 and the control part, it may be confirmed that the sample provided to the test strip 200 has developed and reached the reaction part and the determination part 130, and that the test has been performed normally. Note that the control part is manufactured in the same manner as the above-described determination part 130 and may adopt the same configuration, except that a different type of capture ligand that specifically binds to the labeled antibody 150 is used instead of the capture ligand 131.

[Analyte Measurement Method]

Next, an analyte 160 measurement method according to one embodiment of the invention performed using the test strip 200 will be described.

The analyte 160 measurement method of the present embodiment is an analyte 160 measurement method for detecting or quantifying the analyte 160 included in a sample. The analyte 160 measurement method of the present embodiment uses a test strip 200 including a membrane 110 and a determination part 130 in which a capture ligand 131 that specifically binds to the analyte 160 is immobilized on the membrane 110. The analyte 160 measurement method of the present embodiment may include the following steps (I) to (III):

step (I): a step of bringing the analyte 160 included in the sample into contact with the labeled antibody 150 in which an antibody that specifically binds to the analyte 160 is labeled with a metal-resin composite 100 having a structure in which multiple metal particles 20 are immobilized on the resin particles 10;

step (II): a step of bringing the composite 170 including the analyte 160 and the labeled antibody 150 formed in step (I) into contact with the capture ligand 131 at the determination part 130; and

step (III): a step of measuring color intensity derived from light energy absorption due to localized surface plasmon resonance and/or electron transition of the metal-resin composite 100.

Step (I):

Step (I) is a step of bringing the analyte 160 included in the sample into contact with the labeled antibody 150. The manner of contact is not particularly limited as long as the composite 170 including the analyte 160 and the labeled antibody 150 is formed. For example, the sample may be supplied to the sample addition part 120 or reaction part (not shown) of the test strip 200, and the analyte 160 may be brought into contact with the labeled antibody 150 at the sample addition part 120 or reaction part, or the analyte 160 in the sample may be brought into contact with the labeled antibody 150 before supplying the sample to the test strip 200.

The composite 170 formed in step (I) develops and moves on the test strip 200, and reaches the determination part 130.

Step (II):

Step (II) is a step of bringing the composite 170 including the analyte 160 and the labeled antibody 150, which was formed in step (I), into contact with the capture ligand 131 at the determination part 130 of the test strip 200. In response to bringing the composite 170 into contact with the capture ligand 131, the capture ligand 131 specifically binds to the analyte 160 of the composite 170. As a result, the composite 170 is captured at the determination part 130.

Since the capture ligand 131 does not specifically bind to the labeled antibody 150, in the case of the labeled antibody 150 unbound to the analyte 160 reaching the determination part 130, the labeled antibody 150 unbound to the analyte 160 passes through the determination part 130. Here, in the case of a control part (not shown) having another capture ligand that specifically binds to the labeled antibody 150 fixed thereto being formed on the test strip 200, the labeled antibody 150 that has passed through the determination part 130 continues to develop and binds with the another capture ligand at the control part. As a result, the labeled antibody 150 that has not formed the composite 170 with the analyte 160 is captured at the control part.

After step (II), before step (III) as necessary, a washing step of washing the test strip 200 with a buffer solution commonly used in biochemical tests, such as water, physiological saline, phosphate buffer solution, etc. may be performed. By the washing step, the labeled antibody 150 that has not been captured by the determination part 130, or the determination part 130 and the control part (the labeled antibody 150 that is not bound to the analyte 160 and does not form the composite 170) may be removed.

By performing the washing step, in step (III), the background color intensity may be reduced according to measuring the coloration due to localized surface plasmon resonance and/or light energy absorption by electron transition of the metal-resin composite 100 at the determination part 130, or the determination part 130 and the control part, and the signal/background ratio may be increased to further improve detection sensitivity and quantitativeness.

Step (III):

Step (III) is a step of measuring color intensity derived from light energy absorption by localized surface plasmon resonance and/or electron transition of the metal-resin composite 100. After performing the above step (II) or the washing step as necessary, the color intensity derived from light energy absorption by localized surface plasmon resonance and/or electron transition of the metal-resin composite 100 is measured in the test strip 200.

In the case of a control part being formed on the test strip 200, by step (II), the labeled antibody 150 is captured by another capture ligand at the control part and a composite is formed. Therefore, in step (III), coloration due to light energy absorption by localized surface plasmon resonance and/or electron transition may be generated not only at the determination part 130 but also at the control part in the test strip 200. In this way, by measuring the color intensity at the control part together with the determination part 130, it may be confirmed whether the sample provided to the test strip 200 has normally developed and reached the reaction part and the determination part 130.

<Sample and Analyte>

The sample in the analyte measurement method of the present embodiment is not particularly limited as long as it includes a substance that may serve as an antigen such as protein as the analyte 160. For example, biological samples including the target analyte 160 (i.e. whole blood, serum, plasma, urine, saliva, sputum, nasal or pharyngeal swab fluid, cerebrospinal fluid, amniotic fluid, nipple discharge, tears, sweat, exudate from skin, extracts from tissues, cells, and feces, etc.) and food extracts may be mentioned. As necessary, prior to the above step (I), the analyte 160 included in the sample may be pretreated in order to facilitate the specific binding reaction between the labeled antibody 150 and the capture ligand 131 with the analyte 160. Here, the pretreatment includes chemical treatment using various chemicals such as acids, bases, surfactants, etc. and physical treatment using heating, stirring, ultrasonic waves, etc. Particularly, in the case of the analyte 160 being a substance that is not normally exposed on the surface, such as influenza virus NP antigen, it is preferable to perform treatment with surfactants, etc. As the surfactant used for this purpose, nonionic surfactants may be used in consideration of specific binding reactions, for example, the binding reactivity between the capture ligand 131 and the analyte 160 such as antigen-antibody reactions.

In addition, the sample may be appropriately diluted with solvents (water, physiological saline, or buffer solution, etc.) or water-miscible organic solvents used in conventional immunoassay methods.

The analyte 160 is not particularly limited and known ones may be used, including those with strong anionic properties, those with strong cationic properties, and others. Examples of the analyte 160 include proteins (including polypeptides, oligopeptides, etc.) such as tumor markers, signal transduction substances, hormones, nucleic acids (including single-stranded or double-stranded DNA, RNA, polynucleotides, oligonucleotides, PNA (peptide nucleic acid), etc.) or substances having nucleic acids, sugars (including oligosaccharides, polysaccharides, sugar chains, etc.) or substances having sugar chains, lipids and other molecules, and are not particularly limited as long as they specifically bind to the labeled antibody 150 and the capture ligand 131. Examples include carcinoembryonic antigen (CEA), HER2 protein, prostate-specific antigen (PSA), CA19-9, α-fetoprotein (AFP), immunosuppressive acidic protein (IAP), CA15-3, CA125, estrogen receptor, progesterone receptor, fecal occult blood, troponin I, troponin T, CK-MB, CRP, human chorionic gonadotropin (HCG), luteinizing hormone (LH), follicle-stimulating hormone (FSH), syphilis antibody, influenza virus human hemoglobin, chlamydia antigen, group A β-hemolytic streptococcus antigen, HBs antibody, HBs antigen, rotavirus, adenovirus, albumin, glycated albumin, etc. Among these, antigens that are solubilized by nonionic surfactants are preferable, and antigens that form self-assemblies such as viral nucleoproteins are more preferable.

<Labeled Antibody>

The labeled antibody 150 is used in step (I) to contact with the analyte 160 contained in the sample to form the composite 170 including the analyte 160 and the labeled antibody 150. The labeled antibody 150 is formed by labeling an antibody that specifically binds to the analyte 160 with the metal-resin composite 100 having a structure in which multiple metal particles 20 are immobilized on the resin particles 10. Here, "labeling" means that in steps (I) to (III), the metal-resin composite 100 is fixed to the antibody directly or indirectly by chemical or physical bonding, adsorption, etc. to such an extent that the metal-resin composite 100 does not detach from the labeled antibody 150. For example, the labeled antibody 150 may be formed by directly bonding the metal-resin composite 100 to the antibody, or may be formed by bonding the antibody and the metal-resin composite 100 via any linker molecule, or may be formed by each being fixed to insoluble particles.

Also, in the present embodiment, the "antibody" is not particularly limited and known antibodies may be used, including those with strong anionic properties, those with strong cationic properties, and others. For example, polyclonal antibodies, monoclonal antibodies, antibodies obtained by genetic recombination, and antibody fragments having antigen-binding ability [for example, H chain, L chain, Fab, F(ab')2, etc.] may be used. Also, as immunoglobulins, any of IgG, IgM, IgA, IgE, and IgD may be used. As animal species for antibody production, humans as well as non-human animals (for example, mouse, rat, rabbit, goat, horse, etc.) may be used. Specific examples of antibodies include anti-PSA antibody, anti-AFP antibody, anti-CEA antibody, anti-adenovirus antibody, anti-influenza virus antibody, anti-HCV antibody, anti-IgG antibody, anti-human IgE antibody, and the like.

<Preferred Manufacturing Method of Labeled Antibody>

Next, a preferred manufacturing method of the labeled antibody 150 will be described. The manufacturing of the labeled antibody 150 includes at least the following step A:

step A) A step of obtaining the labeled antibody 150 by mixing and binding the metal-resin composite 100 with an antibody under a first pH condition,
and preferably further include step B:

step B) A step of treating the labeled antibody 150 under a second pH condition.

[Step A]

In step A, the metal-resin composite 100 is mixed with an antibody under a first pH condition to obtain the labeled antibody 150. In step A, it is preferable to bring the solid metal-resin composite 100 into contact with the antibody in a state of being dispersed in a liquid phase.

The first pH condition is preferably within a range of pH 2 to 10, and more preferably within a range of pH 5 to 9, for example, from the viewpoint of uniformly bringing the metal-resin composite 100 into contact with the antibody while maintaining dispersion of the metal-resin composite 100 and activity of the antibody. In the case of conditions for binding the metal-resin composite 100 and the antibody being less than pH 2, the antibody may be denatured and inactivated due to strong acidity, and in the case of exceeding pH 10, aggregation may occur according to mixing the metal-resin composite 100 and the antibody, making dispersion difficult. However, in the case of the antibody not being inactivated by strong acidity, treatment is possible even at less than pH 2.

Step A is preferably performed in a binding buffer solution adjusted to the first pH condition. For example, a predetermined amount of the metal-resin composite 100 is mixed with the binding buffer solution adjusted to the above pH, and thoroughly mixed. As the binding buffer solution, for example, a boric acid solution adjusted to a predetermined concentration may be used. The pH adjustment of the binding buffer solution may be performed using, for example, hydrochloric acid, sodium hydroxide, or the like.

Next, a predetermined amount of antibody is added to the obtained mixed solution, and the mixture is sufficiently stirred and mixed to obtain a labeled antibody-containing solution. From the labeled antibody-containing solution obtained in this manner, only the labeled antibody 150 may be collected as a solid portion by liquid separation means such as centrifugation.

[Step B]

In step B, the labeled antibody 150 obtained in step A is treated under a second pH condition to perform blocking that suppresses non-specific adsorption to the labeled antibody 150. In this case, the labeled antibody 150 collected by the liquid separation means is dispersed in a liquid phase under the second pH condition.

The second pH condition is preferably within a range of pH 2 to 10, for example, from the viewpoint of maintaining antibody activity and suppressing aggregation of the labeled antibody 150, and more preferably within a range of pH 5 to 9 from the viewpoint of suppressing non-specific adsorption of the labeled antibody 150. In the case of blocking conditions being less than pH 2, the antibody may be denatured and inactivated due to strong acidity, and in the case of exceeding pH 10, the labeled antibody 150 aggregates, making dispersion difficult.

Step B is preferably performed using a blocking buffer solution in which a blocking agent is adjusted to the second pH condition. For example, the blocking buffer solution adjusted to the above pH is added to a predetermined amount of the labeled antibody 150, and the labeled antibody 150 is uniformly dispersed in the blocking buffer solution. As the blocking buffer solution, for example, it is preferable to use a solution of protein that does not bind to the substance to be detected. The blocking agent usable in the blocking buffer solution is not particularly limited, and known ones may be used, including those with strong anionic properties, those with strong cationic properties, and others. For example, in the case of proteins, bovine serum albumin, egg white albumin, casein, gelatin, whey, and the like may be mentioned. More specifically, it is preferable to use a bovine serum albumin solution adjusted to a predetermined concentration. The pH adjustment of the blocking buffer solution may be performed using, for example, hydrochloric acid, sodium hydroxide, or the like. For dispersion of the labeled antibody 150, it is preferable to use dispersion means such as ultrasonic treatment. In this manner, a dispersion in which the labeled antibody 150 is uniformly dispersed is obtained.

In the above step A and step B, the metal-resin composite 100 is less likely to undergo pH-induced aggregation and may be treated over a wide pH range from acidic to alkaline. Therefore, the metal-resin composite 100 used in the invention also has the advantage of being less subject to restrictions on the manufacturing conditions of the labeled antibody 150.

As described above, a dispersion of the labeled antibody 150 is obtained. From this dispersion, only the labeled antibody 150 may be collected as a solid portion by liquid separation means such as centrifugation. Additionally, washing treatment, storage treatment, and the like may be performed as necessary. Hereinafter, the washing treatment and storage treatment are described.

(Washing Treatment)

In the washing treatment, a washing buffer solution is added to the labeled antibody 150 collected by the liquid separation means, and the labeled antibody 150 is uniformly dispersed in the washing buffer solution. For dispersion, it is preferable to use dispersion means such as ultrasonic treatment. The washing buffer solution is not particularly limited, but for example, Tris buffer solution, glycinamide buffer solution, arginine buffer solution, and the like at a predetermined concentration adjusted within a pH range of 8 to 9 may be used. The pH adjustment of the washing buffer solution may be performed using, for example, hydrochloric acid, sodium hydroxide, or the like. The washing treatment of the labeled antibody 150 may be repeated multiple times as necessary.

(Storage Treatment)

In the storage treatment, a storage buffer solution is added to the labeled antibody 150 collected by the liquid separation means, and the labeled antibody 150 is uniformly dispersed in the storage buffer solution. For dispersion, it is preferable to use dispersion means such as ultrasonic treatment. As the storage buffer solution, for example, a solution obtained by adding a predetermined concentration of an anti-aggregation agent and/or a stabilizer to the washing buffer solution may be used. As the anti-aggregation agent, for example, saccharides represented by sucrose, maltose, lactose, and trehalose, and polyhydric alcohols represented by glycerin and polyvinyl alcohol may be used. The stabilizer is not particularly limited, but for example, proteins such as bovine serum albumin, egg white albumin, casein, and gelatin may be used. In this manner, the storage treatment of the labeled antibody 150 may be performed.

In each of the above steps, surfactants and preservatives such as sodium azide and paraoxybenzoic acid ester may be further used as necessary.

[Analyte Measurement Kit]

The analyte measurement kit according to one embodiment of the invention is a kit for detecting or quantifying the analyte 160 contained in a sample based on the analyte measurement method of the present embodiment using, for example, the test strip 200.

The analyte measurement kit of the present embodiment includes:

the membrane 110;
the test strip 200 including the determination part 130 in which the capture ligand 131 that specifically binds to the analyte 160 is immobilized on the membrane 110; and
a detection reagent including the labeled antibody 150 in which an antibody that specifically binds to the analyte 160 is labeled with the metal-resin composite 100 having a structure in which multiple metal particles 20 are immobilized on the resin particles 10.

The analyte measurement kit of the present embodiment may further include other components as necessary.

In using the analyte measurement kit according to the present embodiment, after implementing step (I) by bringing the analyte 160 in the sample into contact with the labeled antibody 150 in the detection reagent, the sample may be supplied to reaction part or the sample addition part 120 of the test strip 200, and step (II) and step (III) may be sequentially implemented. Alternatively, after applying the detection reagent upstream of the determination part 130 of the test strip 200 and appropriately drying to form a reaction part, the sample may be added to the formed reaction part or a position upstream of the reaction part (for example, sample addition part 120), and steps (I) to (III) may be sequentially implemented.

Moreover, as applications other than labeling substance for immunological assay or immunological assay reagent of the metal-resin composite 100, it may be preferably applied as solid catalyst, pigment, paint, conductive material, electrode, and sensor element.

Examples

Next, the invention will be specifically described by Examples, but the invention is not limited by these Examples in any way. In the following Examples and Comparative Examples, unless otherwise specified, various measurements and evaluations are based on the following.

<Absorbance Measurement of Metal-Resin Composite Particles>

A metal-resin composite particle dispersion (dispersion medium: water) adjusted to 0.01 wt% was placed in a quartz glass cell (optical path length 10 mm), and absorbance at 570 nm was measured using a spectrophotometer (UV3600, manufactured by Shimadzu Corporation).

<Measurement of Solid Content Concentration and Metal Loading Amount>

1 g of metal-resin composite particle dispersion before concentration adjustment was placed in a porcelain crucible and dried at 70°C for 3 hours. The weight before and after drying was measured, and the solid content concentration was calculated using the following formula.

Solid content concentration (wt%) = [Weight after drying (g)/Weight before drying (g)] $\times$ 100

Furthermore, the Sample after the above drying treatment was further heat-treated at 500°C for 3 hours, the weight before and after heat treatment was measured, and the metal loading amount was calculated using the following formula.

Metal loading amount (wt%) = [Weight after heat treatment (g)/Weight before heat treatment (g)] $\times$ 100

<Measurement of Average Particle Diameter and Particle Size Distribution of Resin Particles and Metal-Resin Composite Particles>

The measurement was performed using a centrifugal sedimentation particle size distribution analyzer (LUMi-Sizer610 manufactured by LUM GmbH). The measurement was performed with the particles dispersed in water or solution.

<Measurement of Average Particle Diameter of Metal Particles and Calculation of Coefficient of Variation (CV Value)>

A substrate created by dropping the metal-resin composite particle dispersion onto a metallic mesh with carbon support film was observed using a field emission scanning electron microscope (FE-SEM; SU-9000, manufactured by Hitachi High-Technologies Corporation), and the area average diameter of arbitrary 100 metal particles was measured from the obtained images to determine the average particle diameter. Additionally, the coefficient of variation (CV value) was calculated by dividing the standard deviation of the particle diameter of the metal particles by the average particle diameter.

<Zeta Potential Measurement>

The zeta potential was measured by electrophoretic light scattering method using Zetasizer Nano-ZS manufactured by Malvern as the measurement device. The Sample was diluted to 0.01 wt% with pure water and adjusted to each pH value in the range of pH 3 to 10 using hydrochloric acid or NaOH aqueous solution as the Measurement Sample. After pH measurement using a pH meter (HORIBA LAQUA twin), zeta potential measurement was performed to measure the behavior of zeta potential changes at multiple pH points from pH 3 to 10. The variation range of zeta potential was calculated from the maximum value of zeta potential in the acidic region of pH 3 to 6 and the minimum value of zeta potential in the alkaline region of pH 8 to 10. Additionally, a linear function connecting an arbitrary zeta potential point greater than 0 mV and an arbitrary zeta potential point less than 0 mV, where the zeta potential is close to 0 mV, was determined, and the pH at which the zeta potential becomes 0 mV, that is, the zero charge point, was calculated.

<Dispersibility Evaluation>

A Sample diluted to 0.01 wt% with pure water was placed 1 mL into a 2 cc tube (AS ONE polypropylene Violamo microtube), left to stand at room temperature for 3 days, and then the precipitate formed was stirred with a vortex mixer (LSE TM manufactured by CORNING) for 10 seconds. The dispersion state after stirring was visually observed and evaluated.

Evaluation (○: all precipitates are dispersed, △: precipitates are dispersed but some remain as precipitates, ×: precipitates are hardly dispersed and remain as precipitates)

[Manufacturing Example 1]

<Synthesis of Resin Particles>

80 wt% trioctylmethylammonium chloride aqueous solution (1.33 g, 2.63 mmol) and 50 wt% polyethylene glycol methyl ether methacrylate aqueous solution (10.00 g, 2.40 mmol) were dissolved in 300 g of pure water, then 2-vinylpyridine (48.00 g, 457 mmol) and divinylbenzene (2.00 g, 15.4 mmol) were added, and the mixture was stirred under nitrogen flow at 30°C for 50 minutes, then at 60°C for 30 minutes. After stirring, 2,2-azobis(2-methylpropionamidine) dihydrochloride (0.250 g, 0.922 mmol) dissolved in 18.00 g of pure water was added dropwise, and the mixture was stirred at 60°C for 3.5 hours to obtain resin particles A-1 with an average particle diameter of 345 nm. The particles were precipitated by centrifugation (9000 rpm, 40 minutes), the supernatant was removed, then redispersed in pure water, and impurities were removed by filtration treatment. Thereafter, concentration adjustment was performed to obtain 10 wt% resin particle dispersion B-1.

[Manufacturing Example 2]

<Synthesis of Resin Particles>

After adding 93.87 g of pure water to B-1 (51.01 g) obtained in Manufacturing Example 1 for dilution, the mixture was stirred at 28°C for 80 minutes while blowing nitrogen. After stirring, methacrylic acid (0.086 g, 0.999 mmol) dissolved in 0.475 g of pure water was added dropwise, and the mixture was stirred at 40°C for 30 minutes. After stirring, the temperature was raised to 70°C, and 2,2-azobis(2-methylpropionamidine) dihydrochloride (0.011 g, 0.041 mmol) dissolved in 0.664 g of pure water was added dropwise, and the mixture was stirred at 70°C for 150 minutes to obtain resin particles A-2 with an average particle diameter of 346 nm. The particles were precipitated by centrifugation (8460 rpm, 30 minutes), the supernatant was removed, then redispersed in pure water, and concentration adjustment was performed to obtain 10 wt% resin particle dispersion B-2. In the resin particles A-2, the molar ratio of cationic functional groups (pyridinyl groups) contained in poly(2-vinylpyridine), which is a polymer having cationic functional groups, to anionic functional groups (carboxyl groups) contained in polymethacrylic acid, which is a polymer having anionic functional groups, was 97.7:2.3.

[Manufacturing Example 3]

<Synthesis of Resin Particles>

After adding 98.54 g of pure water to B-1 (51.01 g) obtained in Manufacturing Example 1 for dilution, the mixture was stirred at 28°C for 80 minutes while blowing nitrogen. After stirring, methacrylic acid (0.257 g, 2.99 mmol) dissolved in 0.476 g of pure water was added dropwise, and the mixture was stirred at 40°C for 30 minutes. After stirring, the temperature was raised to 70°C, and 2,2-azobis(2-methylpropionamidine) dihydrochloride (0.011 g, 0.041 mmol) dissolved in 0.665 g of pure water was added dropwise, and the mixture was stirred at 70°C for 150 minutes to obtain resin particles A-3 with an average particle diameter of 347 nm. The particles were precipitated by centrifugation (8460 rpm, 30 minutes), the supernatant was removed, then redispersed in pure water, and concentration adjustment was performed to obtain 10 wt% resin particle dispersion B-3. In the resin particles A-3, the molar ratio of cationic functional groups (pyridinyl groups) contained in poly(2-vinylpyridine), which is a polymer having cationic functional groups, to anionic functional groups (carboxyl groups) contained in polymethacrylic acid, which is a polymer having anionic functional groups, was 93.4:6.6.

[Manufacturing Example 4]

<Preparation of Buffer>

After dissolving 15.14 g (125 mmol) of 2-amino-2-(hydroxymethyl)-1,3-propanediol in 500 g of pure water, 26.66 g (125 mmol) of 2-morpholinoethanesulfonic acid monohydrate was added and dissolved by stirring. Subsequently, filtration was performed to obtain Buffer 1.

[Manufacturing Example 5]

<Synthesis of Resin Particles>

After dissolving 80 wt% trioctylmethylammonium chloride aqueous solution (1.50 g, 2.96 mmol) and 50 wt% polyethylene glycol methyl ether methacrylate aqueous solution (10.00 g, 4.50 mmol) in 300 g of pure water, 2-vinylpyridine (48.00 g, 457 mmol) and divinylbenzene (2.00 g, 15.4 mmol) were added, and the mixture was stirred under nitrogen flow at 30°C for 50 minutes, then at 60°C for 30 minutes. After stirring, 2,2-azobis(2-methylpropionamidine) dihydrochloride (0.250 g, 0.922 mmol) dissolved in 18.00 g of pure water was added dropwise, and the mixture was stirred at 60°C for 3.5 hours. Thereafter, 20 g (250 mmol) of sodium hydroxide aqueous solution with concentration of 50 wt% was added, and the mixture was stirred at 60°C for 2 hours to obtain resin particles A-4 with an average particle diameter of 312 nm. The particles were precipitated by centrifugation (9000 rpm, 40 minutes), the supernatant was removed, then redispersed in pure water, and impurities were removed by dialysis treatment. Thereafter, concentration adjustment was performed to obtain 10 wt% resin particle dispersion B-4.

[Manufacturing Example 6]

<Synthesis of Resin Particles>

Resin particles A-5 with an average particle diameter of 439 nm and 10 wt% resin particle dispersion B-5 were obtained by the same method as Manufacturing Example 1, except that 0.39 g (0.77 mmol) of 80 wt% trioctylmethylammonium chloride aqueous solution and 0.50 g (1.84 mmol) of 2,2-azobis(2-methylpropionamidine) dihydrochloride were used.

[Example 1]

<Synthesis of Gold-Resin Composite Particles>

After adding 75 g of pure water to B-2 (30.00 g) obtained in Manufacturing Example 2, 7 wt% chloroauric acid aqueous solution (42.91 g) was added, and the mixture was stirred at 30°C for 3 hours. This mixed solution was centrifuged (3000 rpm, 15 minutes), and the supernatant was removed to remove excess chloroauric acid. Thereafter, the concentration was adjusted to obtain 5 wt% gold ion-adsorbed resin particle dispersion C-1.

Next, 473.00 g of buffer 1 was added to 4257.48 g of pure water, and hydrochloric acid of concentration 1M was added while stirring until the pH reached 6.0. Subsequently, 100.00 g of 1 wt% Triton X-100 aqueous solution was added, and a reducing agent solution was prepared by adding 528 mM dimethylamine borane aqueous solution (48 mL) while stirring at 3°C. Thereafter, a diluted dispersion prepared by adding 65.00 g of pure water to C-1 (65.00 g) was added dropwise to this reducing agent solution over 13 minutes while stirring at 3°C, followed by stirring at 3°C for 80 minutes and at room temperature for 3 hours to obtain gold-resin composite particles D-1 with an average particle diameter of 348 nm. After concentrating D-1 by centrifugation, it was purified by dialysis treatment, and the concentration was adjusted to obtain 1 wt% gold-resin composite particle dispersion E-1. The absorbance of gold-resin composite particles F-1 in E-1 was 1.43. Moreover, the average particle diameter of gold particles in F-1 was 7.3 nm, the CV value was 0.36, and the gold loading amount was 49.4 wt%. The results of zeta potential measurement of F-1 are shown in Table 1.

In these gold-resin composite particles F-1, the gold particles include at least inclusion gold particles completely encapsulated in the resin particles, and partially exposed gold particles having portions embedded within the resin particles and portions exposed outside the resin particles, and at least some of the gold particles were three-dimensionally distributed in the resin particles. The gold particles were 97% present within a range of 40% of the particle radius in the depth direction from the surface of the resin particles.

[Example 2]

<Synthesis of Gold-Resin Composite Particles>

After adding 26 g of pure water to B-3 (30.00 g) obtained in Manufacturing Example 3, 7 wt% chloroauric acid aqueous solution (14.30 g) was added, and the mixture was stirred at 30°C for 3 hours. This mixed solution was centrifuged (3000 rpm, 15 minutes), and the supernatant was removed to remove excess chloroauric acid. Thereafter, the concentration was adjusted to obtain 5 wt% gold ion-adsorbed resin particle dispersion C-2.

Next, 4.73 g of buffer 1 was added to 42.57 g of pure water, and hydrochloric acid of concentration 1M was added while stirring until the pH reached 6.0. Subsequently, 1.00 g of 1 wt% Triton X-100 aqueous solution was added, and a reducing agent solution was prepared by adding 528 mM dimethylamine borane aqueous solution (0.48 mL) while stirring at 3°C. Thereafter, a diluted dispersion prepared by adding 0.65 g of pure water to C-2 (0.65 g) was added dropwise to this reducing agent solution over 5 minutes while stirring at 3°C, followed by stirring at 3°C for 60 minutes and at room temperature for 60 minutes to obtain gold-resin composite particles D-2 having an average particle diameter of 348 nm. After concentrating D-2 by centrifugation, it was purified by dialysis treatment, and the concentration was adjusted to obtain 1 wt% gold-resin composite particle dispersion E-2. The absorbance of the gold-resin composite particles F-2 in E-2 was 1.39. In addition, the average particle diameter of the gold particles in F-2 was 8.7 nm, the CV value was 0.41, and the gold loading amount was 54.4 wt%. The results of zeta potential measurement of F-2 are shown in Table 1.

In these gold-resin composite particles F-2, the gold particles include at least inclusion gold particles completely encapsulated in the resin particles, and partially exposed gold particles having portions embedded within the resin particles and portions exposed outside the resin particles, and at least some of the gold particles were three-dimensionally distributed in the resin particles. The gold particles were 97% present within a range of 40% of the particle radius in the depth direction from the surface of the resin particles.

[Comparative Example 1]

<Synthesis of Gold-Resin Composite Particles>

After adding 255 g of pure water to B-4 (91.5 g) obtained in Manufacturing Example 5, 400 mM chloroauric acid aqueous solution (147 g) was added, and the mixture was stirred at room temperature for 3 hours. This mixed solution was centrifuged (3000 rpm, 30 minutes), and the supernatant was removed to remove excess chloroauric acid. Thereafter, the concentration was adjusted to obtain 2.5 wt% gold ion-adsorbed resin particle dispersion C-3.

Next, C-3 (43.3 g) was added to 1580 g of pure water, and 528 mM dimethylamine borane aqueous solution (10.0 g) was added dropwise over 2 minutes while stirring at 3°C, followed by stirring at 3°C for 1 hour and at room temperature for 3 hours to obtain gold-resin composite particles D-3 having an average particle diameter of 322 nm. After concentrating D-3 by centrifugation, it was purified by dialysis treatment, and the concentration was adjusted to obtain 1 wt% gold-resin composite particle dispersion E-3. The absorbance of the gold-resin composite particles F-3 in E-3 was 1.27. In addition, the average particle diameter of the gold particles in F-3 was 30 nm, the CV value was 0.49, and the gold loading amount was 53.8 wt%. The results of zeta potential measurement of F-3 are shown in Table 1.

In these gold-resin composite particles F-3, the gold particles include at least inclusion gold particles completely encapsulated in the resin particles, and partially exposed gold particles having portions embedded within the resin particles and portions exposed outside the resin particles, and at least some of the gold particles were three-dimensionally distributed in the surface layer part of the resin particles. The gold particles were 97% present within a range of 40% of the particle radius in the depth direction from the surface of the resin particles.

[Comparative Example 2]

<Gold-Resin Composite Particle Synthesis>

After adding 255 g of pure water to B-5 (91.5 g) obtained in Manufacturing Example 6, 400 mM chloroauric acid aqueous solution (147 g) was added, and the mixture was stirred at room temperature for 3 hours. This mixed solution was centrifuged (3000 rpm, 30 minutes), and the supernatant was removed to remove excess chloroauric acid. Thereafter, the concentration was adjusted to obtain 2.5 wt% gold ion-adsorbed resin particle dispersion C-4.

Next, C-4 (43.3 g) was added to 1580 g of pure water, and 528 mM dimethylamine borane aqueous solution (10.0 g) was added dropwise over 2 minutes while stirring at 3°C, followed by stirring at 3°C for 1 hour and at room temperature for 3 hours to obtain gold-resin composite particles D-4 having an average particle diameter of 322 nm. After concentrating D-4 by centrifugation, it was purified by dialysis treatment, and the concentration was adjusted to obtain 1 wt% gold-resin composite particle dispersion E-4. The absorbance of the gold-resin composite particles F-4 in E-4 was 1.27. In addition, the average particle diameter of the gold particles in F-4 was 30 nm, the CV value was 0.31, and the gold loading amount was 53.8 wt%. In these gold-resin composite particles F-4, the gold particles include at least inclusion gold particles completely encapsulated in the resin particles, and partially exposed gold particles having portions embedded within the resin particles and portions exposed outside the resin particles, and at least some of the gold particles were three-dimensionally distributed in the surface layer part of the resin particles. The gold particles were 97% present within a range of 40% of the particle radius in the depth direction from the surface of the resin particles.

<Mercaptopropionic Acid Treatment>

E-4 (0.1 g) and 10 mM mercaptopropionic acid aqueous solution (1.0 g) were mixed and stirred at 23°C for 1 hour. After precipitation by centrifugation (12000 rpm, 5 minutes) and removal of the supernatant, pure water (1.0 g) was added for redispersion to remove excess mercaptopropionic acid, thereby producing mercaptopropionic acid-treated gold-resin composite particles F-4A.

The average particle diameter of F-4A was 455 nm, and the absorbance was 1.36. In addition, the average particle diameter of the gold particles in F-4A was 20 nm, and the gold loading amount was 48.3 wt%. The results of zeta potential measurement of F-4A are shown in Table 1.

Mercaptopropionic acid-treated gold-resin composite particle dispersion E-4A was prepared by dispersing the mercaptopropionic acid-treated gold-resin composite particles F-4A in pure water.

[Comparative Example 3]

<Polyglutamic Acid Treatment>

E-4 (0.1 g) obtained in Comparative Example 2 and 0.5 wt% polyglutamic acid aqueous solution (1.0 g) were mixed and stirred at 23°C for 1 hour. After precipitation by centrifugation (12000 rpm, 5 minutes) and removal of the supernatant, pure water (1.0 g) was added to remove excess polyglutamic acid, thereby producing polyglutamic acid-treated gold-resin composite particles F-4B. The results of zeta potential measurement of F-4B are shown in Table 1.
Polyglutamic acid-treated gold-resin composite particle dispersion E-4B was prepared by dispersing the polyglutamic acid-treated gold-resin composite particles F-4B in pure water.

[Table 1]

| | Gold-resin composite | Zeta potential [mV] | | | Zero charge point [pH] | Dispersibility |
| --- | --- | --- | --- | --- | --- | --- |
| | | Maximum value | Minimum value | Variation range | | |
| Example 1 | F-1 | 38.5 | -41.2 | 79.7 | 7.2 | O |
| Example 2 | F-2 | 37.0 | -44.5 | 81.5 | 6.4 | O |
| Comparative Example 1 | F-3 | 46.1 | -16.9 | 63.0 | 8.0 | △ |
| Comparative Example 2 | F-4A | 41.5 | -36.3 | 77.8 | 6.0 | × |
| Comparative Example 3 | F-4B | 23.8 | -51.3 | 75.1 | 5.6 | × |

From Table 1, Examples 1, 2 and Comparative Examples 2, 3 had larger absolute values of the maximum value and minimum value of zeta potential and larger variation ranges compared to Comparative Example 1 which underwent alkali treatment. Moreover, Examples 1 and 2 showed good dispersibility in pure water, but Comparative Examples 2 and 3, which underwent mercaptopropionic acid treatment and polyglutamic acid treatment, were confirmed to have poor dispersibility due to precipitation. The reason for this is considered to be that in mercaptopropionic acid treatment and polyglutamic acid treatment, molecules containing carboxyl groups were merely adsorbed on the surface of the gold-resin composite and thus easily desorbed, reducing dispersibility in pure water. In contrast, in Examples 1 and 2, the resin particles have polymethacrylic acid polymerized on the surface or inside of poly(2-vinylpyridine), which is a polymer having cationic functional groups, making it difficult for carboxyl groups, which are anionic functional groups, to desorb from the resin particles. This is considered to contribute to good dispersibility in pure water and, as shown in the test results later, to suppression of clogging in immunochromatography.

[Manufacturing Example 6]

<Preparation of Binding Buffer Solution>

A 0.1 mol/L free acid solution of 2-[4-(2-hydroxyethyl)-1-piperazinyl]ethanesulfonic acid (HEPES) was prepared, and pure water and 0.1 mol/L sodium hydroxide solution were mixed to adjust the buffering agent concentration and pH to 7.0, thereby preparing HEPES buffer solution.

[Example 3]

(Binding Step)

After mixing 25 μg of anti-CRP antibody with 0.45 mL of 20 mM HEPES buffer solution (pH 7), 0.05 mL of the 1 wt% gold-resin composite particle dispersion E-1 prepared in Example 1 was added, and inversion stirring was performed at room temperature for 2 hours to obtain labeled antibody dispersion G-1 containing anti-CRP antibody labeled with the gold-resin composite particles F-1.

(Blocking Step)

Next, the labeled antibody dispersion G-1 was centrifuged at 3000 rpm for 5 minutes, and after removing the supernatant, 0.5 mL of 5 mM Tris aqueous solution (pH 5) containing 1 wt% BSA was added to the precipitated sediment, and after ultrasonic dispersion, inversion stirring was further performed at room temperature for 2 hours to obtain labeled antibody dispersion H-1.

(Washing Treatment)

Next, the labeled antibody dispersion H-1 was centrifuged at 3000 rpm for 5 minutes, and after removing the supernatant, 0.5 mL of 5 mM Tris aqueous solution (pH 8.5) containing less than 0.1 wt% surfactant was added to the precipitated sediment, and ultrasonic dispersion was performed. This operation was repeated 3 times as the washing treatment.

(Storage Treatment)

Next, after ice cooling, centrifugation was performed at 3000 rpm for 5 minutes, and after removing the supernatant, 0.5 mL of 5 mM Tris aqueous solution (pH 8.5) containing less than 0.1 wt% surfactant and 10 wt% sucrose was added to the precipitated sediment, and ultrasonic dispersion was performed to obtain labeled antibody dispersion I-1.

(Conjugate Pad Manufacturing)

0.12 mL of the labeled antibody dispersion I-1 was centrifuged at 3000 rpm for 5 minutes, and after removing the supernatant, 0.333 mL of aqueous solution (pH 8.0) containing 5 wt% sucrose and 2.5 wt% BSA was added to the precipitated sediment, and ultrasonic dispersion was performed to obtain labeled antibody dispersion J-1. After uniformly impregnating glass fiber nonwoven fabric with the labeled antibody dispersion J-1, drying was performed at 50°C for 1 hour to manufacture conjugate pad K-1. At this time, the liquid amount of the labeled antibody dispersion J-1 was adjusted so that the content of the gold-resin composite particles F-1 in the conjugate pad K-1 would be 3 μg per test for evaluation by the immunochromatography method described later.

(Manufacturing of Immunochromatographic Strip)

An immunochromatographic strip having the structure shown in FIG. 4 was manufactured. First, anti-CRP antibody was applied to a nitrocellulose membrane 4 having a width of 25 mm to draw a test line 5. Also, anti-mouse IgG antibody was applied to the downstream side of the test line 5 to draw a control line 6. After drying this nitrocellulose membrane 4 at 50°C for 1 hour, a backing sheet 1, the conjugate pad K-1 as a conjugate pad 3, a sample pad 2 (glass fiber nonwoven fabric), and an absorption pad 7 (cotton nonwoven fabric) were laminated as shown in the cross-sectional diagram of the immunochromatographic strip shown in FIG. 4. Finally, the strip was cut to a width of 3.5 mm to manufacture immunochromatographic strip L-1.

(Preparation of Development Solution)

An aqueous solution (pH 7.1) containing 50 mM Tris, 150 mM NaCl, 1.0 wt% BSA, and 1.0 wt% PEG cetyl ether was prepared and used as development solution M-1.

(Evaluation by Immunochromatography Method)

By diluting CRP antigen using the development solution M-1, specimen solutions of positive control (antigen concentration 312 ng/ml, 3.12 ng/ml) and negative control (antigen-free) were prepared. 50 μl of specimen solution was dropped onto the sample pad 2 of the immunochromatographic strip L-1. After 30 minutes had elapsed, the color intensity of the test line 5 was measured with an immunochromatography reader (Hamamatsu Photonics C10066-10). Also, visual determination was made as to whether the gold-resin composite particles F-1 (or labeled antibody) were clogged at the interface between the conjugate pad 3 and the nitrocellulose membrane 4 and on the downstream side thereof. The evaluation results by the immunochromatography method are shown in Table 2.

(Calculation of Bound Antibody Amount)

Using the supernatant removed by centrifugation in the above blocking step, the antibody amount bound to the gold-resin composite particles F-1 was calculated. The calculation of bound antibody amount was performed by measuring the supernatant using a spectrophotometer, determining the antibody amount contained in the supernatant from the absorbance at wavelength 280 nm, and subtracting the antibody amount in the supernatant from the antibody amount of 25 μg used in the binding step. The calculated bound antibody amount and antibody binding yield are shown in Table 2. The binding yield means the weight percentage (%) of attached antibody relative to the total antibody amount used.

[Example 4, Comparative Examples 4 to 6]

In addition to using the gold-resin composite particle dispersion and binding buffer solution shown in Table 2, the binding step, blocking step, washing treatment, storage treatment, conjugate pad preparation, immunochromatographic strip preparation, immunochromatographic evaluation, and calculation of bound antibody amount and antibody binding yield were performed in the same manner as Example 3. Table 2 shows the calculation results of bound antibody amount and antibody binding yield, and the immunochromatographic evaluation results.

From Table 2, based on the test results of Examples 3 to 4 and Comparative Examples 4 to 6, it may be seen that the gold-resin composite particles of the invention suppress clogging in the immunochromatographic evaluation results, obtain good immunochromatographic results without defects, increase the color intensity of the test line, and improve the sensitivity of immunochromatography.

[Table 2]

|  | Gold-resin composite particle dispersion | Binding buffer solution | Bound antibody amount [$\mu$g] | Antibody binding yield [%] | Immunochromatographic evaluation results | | | |
|---|---|---|---|---|---|---|---|---|
|  |  |  |  |  | Clogging | 312 ng/mL | 3.12 ng/mL | 0 ng/mL |
| Example 3 | E-1 | 20mM HEPES buffer solution (pH7) | 18.2 | 72.8 | No | 816.5 | 225.3 | 0.0 |
| Example 4 | E-2 | 20mM HEPES buffer solution (pH7) | 17.8 | 71.3 | No | 802.5 | 210.3 | 0.0 |
| Comparative Example 4 | E-3 | 20mM HEPES buffer solution (pH7) | 17.1 | 68.3 | Yes | 634.6 | 151.8 | 5.5 |
| Comparative Example 5 | E-4A | 20mM HEPES buffer solution (pH7) | 19.8 | 79.1 | Yes | 587.4 | 125.6 | 8.2 |
| Comparative Example 6 | E-4B | 20mM HEPES buffer solution (pH7) | 14.8 | 59.2 | Yes | 456.1 | 83.7 | 6.8 |

Although the embodiments of the invention have been described in detail for illustrative purposes above, the invention is not limited to the above embodiments.

This application claims priority based on Japanese Patent Application No. 2023-051708 filed in Japan on March 28, 2023, and the entire contents of that application are incorporated herein by reference.

Reference Signs List

1...Backing sheet, 2...Sample pad, 3...Conjugate pad, 4...Nitrocellulose membrane, 5... Test line, 6...Control line, 7...Absorption pad, 10...Resin particle, 20...Metal particle, 30...Inclusion particle, 40...Partially exposed particle, 50...Surface adsorbed particle, 60...Surface layer part, 100...Metal-resin composite, 110...Membrane, 120...Sample addition part, 130...Determination part, 131...Capture ligand, 140...Liquid suction part, 150...Labeled antibody, 160...Analyte, 170...Composite, 200...Test strip

**Claims**

1.    A metal-resin composite, comprising:

a resin particle; and
a plurality of metal particles immobilized on the resin particle,
wherein in a range of pH 3 to pH 6, a maximum value of zeta potential is 5 mV or more,

in a range of pH 8 to pH 10, a minimum value of zeta potential is -20 mV or less,
a zero charge point of zeta potential exists in a range of pH 6.1 to pH 9.0, and
the metal particles are particles of gold or an alloy thereof, and a CV value (Coefficient of Variation) of a particle diameter is 0.45 or less.

2. The metal-resin composite according to claim 1,

wherein the resin particle comprises a first polymer and a second polymer of a different type from the first polymer, and
one of the first polymer and the second polymer is a polymer having a cationic functional group, and the other is a polymer having an anionic functional group.

3. The metal-resin composite according to claim 2,
wherein the first polymer is a polymer having a cationic functional group, the second polymer is a polymer having an anionic functional group, and a molar ratio of the cationic functional group included in the first polymer to the anionic functional group included in the second polymer is within a range of 99.5:0.5 to 80:20 as the cationic functional group included in the first polymer:the anionic functional group included in the second polymer.

4. The metal-resin composite according to claim 3,
wherein the first polymer is a polymer having a substituent capable of adsorbing metal ions in its structure.

5. The metal-resin composite according to claim 1,
wherein an average particle diameter of the metal particles is within a range of 1 nm to 100 nm.

6. The metal-resin composite according to claim 1,
wherein the metal particles are formed by reducing metal ions adsorbed to the resin particle with a reducing agent solution in which a buffer solution adjusted to a pH of 3 to 8 and a reducing agent are mixed.

7. The metal-resin composite according to claim 1,
wherein an average particle diameter of the metal-resin composite is within a range of 30 nm to 1000 nm.

8. A labeling substance comprising the metal-resin composite according to claim 1.

9. The labeling substance according to claim 8, which is used by adsorbing an antigen or an antibody to a surface of the metal-resin composite.

10. An immunological assay method using the labeling substance according to claim 8.

11. An immunological assay reagent comprising the labeling substance according to claim 8.

12. An analyte measurement method for detecting or quantifying an analyte contained in a sample, the method comprising:
using a test strip for lateral flow chromatography including a membrane and a determination part in which a capture ligand that specifically binds to the analyte is immobilized on the membrane, including following steps (I) to (III):

step (I): a step of bringing the analyte contained in the sample into contact with a labeled antibody in which an antibody that specifically binds to the analyte is labeled with a metal-resin composite;
step (II): a step of bringing the composite including the analyte and the labeled antibody formed in step (I) into contact with the capture ligand at the determination part; and
step (III): a step of measuring color intensity derived from light energy absorption due to localized surface plasmon resonance and/or electron transition of the metal-resin composite,
wherein the metal-resin composite has a resin particle and a plurality of metal particles immobilized on the resin particle, a maximum value of zeta potential is 5 mV or more in a range of pH 3 to pH 6, a minimum value of zeta potential is -20 mV or less in a range of pH 8 to pH 10, a zero charge point of zeta potential exists in a range of pH 6.1 to pH 9.0, the metal particles are particles of gold or an alloy thereof, and a CV value (Coefficient of Variation) of a particle diameter thereof is 0.45 or less.

13. An analyte measurement kit for detecting or quantifying an analyte contained in a sample, the kit comprising:

a test strip for lateral flow chromatography including a membrane and a determination part in which a capture ligand that specifically binds to the analyte is immobilized on the membrane; and

a detection reagent including a labeled antibody in which an antibody that specifically binds to the analyte is labeled with a metal-resin composite,

wherein the metal-resin composite has a structure in which a plurality of metal particles are immobilized on the resin particle, a maximum value of zeta potential is 5 mV or more in a range of pH 3 to pH 6, a minimum value of zeta potential is -20 mV or less in a range of pH 8 to pH 10, a zero charge point of zeta potential exists in a range of pH 6.1 to pH 9.0, the metal particles are particles of gold or an alloy thereof, and a CV value (Coefficient of Variation) of a particle diameter thereof is 0.45 or less.

14. A test strip for lateral flow chromatography for detecting or quantifying an analyte contained in a sample, the strip comprising:

a membrane;

a determination part in which a capture ligand that specifically binds to the analyte is immobilized on the membrane in a direction in which the sample develops; and

a reaction part including a labeled antibody in which an antibody that specifically binds to the analyte is labeled with a metal-resin composite, located upstream side of the determination part,

wherein the metal-resin composite has a structure in which a plurality of metal particles are immobilized on a resin particle, a maximum value of zeta potential is 5 mV or more in a range of pH 3 to pH 6, a minimum value of zeta potential is -20 mV or less in a range of pH 8 to pH 10, a zero charge point of zeta potential exists in a range of pH 6.1 to pH 9.0, the metal particles are particles of gold or an alloy thereof, and a CV value (Coefficient of Variation) of a particle diameter thereof is 0.45 or less.

FIG. 1

FIG. 2A

FIG. 2B

FIG. 3

FIG. 4

## INTERNATIONAL SEARCH REPORT

| | International application No. |
|---|---|
| | **PCT/JP2024/011542** |

| | | |
|---|---|---|
| **A.** | **CLASSIFICATION OF SUBJECT MATTER** | |

*G01N 33/543*(2006.01)i; *G01N 21/78*(2006.01)i; *G01N 33/553*(2006.01)i
FI:   G01N33/543 595; G01N33/553; G01N33/543 521; G01N21/78 C

According to International Patent Classification (IPC) or to both national classification and IPC

| | | |
|---|---|---|
| **B.** | **FIELDS SEARCHED** | |

Minimum documentation searched (classification system followed by classification symbols)

G01N33/543; G01N21/78; G01N33/553

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); Scopus

| | | |
|---|---|---|
| **C.** | **DOCUMENTS CONSIDERED TO BE RELEVANT** | |

| Category* | Citation of document, with indication, where appropriate, of the relevant passages | Relevant to claim No. |
|---|---|---|
| A | WO 2018/123952 A1 (NIPPON STEEL & SUMIKIN CHEMICAL CO., LTD.) 05 July 2018 (2018-07-05) <br> claim 1, paragraphs [0126], [0133]-[0137], [0149], table 1 | 1-14 |
| A | JP 2012-13687 A (HITACHI MAXELL LTD.) 19 January 2012 (2012-01-19) <br> claim 4, paragraphs [0121], [0132], [0138], fig. 4 | 1-14 |
| A | JP 2019-120497 A (NIPPON STEEL CHEMICAL & MATERIAL CO., LTD.) 22 July 2019 (2019-07-22) <br> paragraphs [0100], [0102], [0104], [0106] | 1-14 |
| A | WO 2022/148777 A1 (THE REGENTS OF THE UNIVERSITY OF MICHIGAN) 14 July 2022 (2022-07-14) <br> entire text, all drawings | 1-14 |
| A | JP 2009-192270 A (BL K.K.) 27 August 2009 (2009-08-27) <br> entire text, all drawings | 1-14 |

☑ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **15 April 2024** | **07 May 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**

| International application No. |
|---|
| **PCT/JP2024/011542** |

### C. DOCUMENTS CONSIDERED TO BE RELEVANT

| Category* | Citation of document, with indication, where appropriate, of the relevant passages | Relevant to claim No. |
|---|---|---|
| A | JP 2009-168495 A (SEKISUI CHEMICAL CO., LTD.) 30 July 2009 (2009-07-30)<br>entire text, all drawings | 1-14 |
| A | WO 2016/002742 A1 (NIPPON STEEL & SUMIKIN CHEMICAL CO., LTD.) 07 January 2016 (2016-01-07)<br>entire text, all drawings | 1-14 |

Form PCT/ISA/210 (second sheet) (July 2022)

**INTERNATIONAL SEARCH REPORT**
Information on patent family members

| International application No. |
| --- |
| **PCT/JP2024/011542** |

| Patent document cited in search report | | | Publication date (day/month/year) | Patent family member(s) | | | Publication date (day/month/year) |
| --- | --- | --- | --- | --- | --- | --- | --- |
| WO | 2018/123952 | A1 | 05 July 2018 | US 2019/0367694 A1 claim 1, paragraphs [0168], [0178]-[0184], [0198], table 1 EP 3564675 A1 CN 110140053 A KR 10-2019-0101992 A | | | |
| JP | 2012-13687 | A | 19 January 2012 | US 2012/0220048 A1 claim 4, paragraphs [0184], [0196], [0201], fig. 4 | | | |
| JP | 2019-120497 | A | 22 July 2019 | (Family: none) | | | |
| WO | 2022/148777 | A1 | 14 July 2022 | CN 116710781 A KR 10-2023-0129266 A | | | |
| JP | 2009-192270 | A | 27 August 2009 | (Family: none) | | | |
| JP | 2009-168495 | A | 30 July 2009 | (Family: none) | | | |
| WO | 2016/002742 | A1 | 07 January 2016 | US 2017/0219574 A1 entire text, all drawings EP 3165560 A1 CN 106661238 A | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009168495 A
- WO 2016002742 A
- WO 2018123952 A
- JP 2023 A
- JP 051708 A

**Non-patent literature cited in the description**

- **K.AKAMATSU** ; **M.SHIMADA** ; **T.TSURUOKA** ; **H.NAWAFUNE** ; **S.FUJII** ; **Y.NAKAMURA**. *Langmuir*, 2010, vol. 26, 1254-1259